(19) **Europäisches Patentamt**
**European Patent Office**
**Office européen des brevets**

(11) **EP 4 542 553 A1**

(12) **EUROPEAN PATENT APPLICATION**

(43) Date of publication:
**23.04.2025 Bulletin 2025/17**

(21) Application number: **24206977.1**

(22) Date of filing: **16.10.2024**

(51) International Patent Classification (IPC):
**G16C 20/20** (2019.01)    **G16C 20/70** (2019.01)
**H01J 49/00** (2006.01)    **G01N 33/68** (2006.01)

(52) Cooperative Patent Classification (CPC):
**G16C 20/70; G01N 33/6848; G16C 20/20;
H01J 49/0031;** G06N 3/092

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.10.2023 US 202318487306**

(71) Applicant: **Thermo Finnigan LLC
San Jose, CA 95134 (US)**

(72) Inventor: **REMES, Philip
San Jose, 95134 (US)**

(74) Representative: **Boult Wade Tennant LLP
Salisbury Square House
8 Salisbury Square
London EC4Y 8AP (GB)**

(54) **METHOD USING REINFORCEMENT LEARNING TO CONTROL A MASS SPECTROMETER**

(57) Embodiments herein relate to neural network control of mass spectrometry processes. A system can comprise a memory that stores, and a processor that executes, computer executable components. The computer executable components can comprise an acquisition component that acquires data for a compound, the data defining a first mass spectrometry spectrum for the compound, an evaluation component that, based on the data, and employing a neural network that is trained on an input dataset comprising an acquisition metric, and employing an associated score that is associated with the acquisition metric, generates a recommendation to perform a mass spectrometry action for the compound, and an execution component that, based on the recommendation, directs execution of the mass spectrometry action at a mass spectrometer and obtaining a mass spectrum result.

EP 4 542 553 A1

# Description

## BACKGROUND

Scientific instruments for use in material analysis can aid in determining the makeup and properties of an unknown composition. In one or more examples, a scientific instrument providing mass spectrometry (MS) analysis or analyzing a result of an MS device can aid in the identification of an unknown composition by aiding in providing or understanding one or more spectra comparing mass calculations based on detection of molecular mass-to-charge ratios after ionization.

## BRIEF DESCRIPTION OF THE DRAWINGS

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.

FIG. 1 illustrates a block diagram of an example scientific instrument for performing operations, in accordance with one or more embodiments described herein.

FIG. 2 illustrates a flow diagram of an example method of performing operations using the scientific instrument of FIG. 1, in accordance with one or more embodiments described herein.

FIG. 3 illustrates a graphical user interface (GUI) that can be used in the performance of one or more of the methods described herein, in accordance with one or more embodiments described herein.

FIG. 4 illustrates a block diagram of an example computing device that can perform one or more of the methods disclosed herein, in accordance with one or more embodiments described herein.

FIG. 5 illustrates a block diagram of an example, non-limiting system that can facilitate reinforcement learning-based mass spectrometry control, in accordance with one or more embodiments described herein.

FIG. 6 illustrates a block diagram of another example, non-limiting system that can facilitate reinforcement learning-based mass spectrometry control, in accordance with one or more embodiments described herein.

FIG. 7 illustrates exemplary high-level processes performed by the material analysis system of FIG. 6, in accordance with one or more embodiments described herein.

FIG. 8 provides an exemplary scoring matrix illustrating inputs employed by the material analysis system of FIG. 6, in accordance with one or more embodiments described herein. FIG. 8 also provides an exemplary scoring key to define the scores of the exemplary scoring matrix, in accordance with one or more embodiments described herein.

FIG. 9 illustrates a set of exemplary reward functions that can be employed by the material analysis system of FIG. 5, in accordance with one or more embodiments described herein.

FIG. 10 illustrates a flow diagram of one or more processes that can be performed by the material analysis system of FIG. 6, in accordance with one or more embodiments described herein.

FIG. 11 illustrates a continuation of the flow diagram of FIG. 10 of one or more processes that can be performed by the material analysis system of FIG. 6, in accordance with one or more embodiments described herein.

FIG. 12 illustrates a flow diagram of one or more processes that can be performed by the material analysis system of FIG. 6, in accordance with one or more embodiments described herein.

FIG. 13 illustrates a continuation of the flow diagram of FIG. 12 of one or more processes that can be performed by the material analysis system of FIG. 6, in accordance with one or more embodiments described herein.

FIG. 14 illustrates a flow diagram of one or more processes that can be performed by the material analysis system of FIG. 6, in accordance with one or more embodiments described herein.

FIG. 15 illustrates a continuation of the flow diagram of FIG. 14 of one or more processes that can be performed by the material analysis system of FIG. 6, in accordance with one or more embodiments described herein.

FIG. 16 illustrates a block diagram of example scientific instrument system in which one or more of the methods described herein can be performed, in accordance with one or more embodiments described herein.

FIG. 17 illustrates a block diagram of an example operating environment into which embodiments of the subject matter described herein can be incorporated.

FIG. 18 illustrates an example schematic block diagram of a computing environment with which the subject matter described herein can interact and/or be implemented at least in part.

## SUMMARY

The following presents a summary to provide a basic understanding of one or more embodiments described herein. This summary is not intended to identify key or critical elements, and/or to delineate scope of particular embodiments or scope of claims. Its sole purpose is to present concepts in a simplified form as a prelude to the more detailed description that is presented later. In one or more embodiments, systems, computer-implemented methods, apparatuses and/or computer program products described herein can provide a process to train and to employ a neural network using reinforcement learning-based training to control a mass spectrometry-based experiment.

In accordance with an embodiment, a system can comprise a memory that stores computer executable components and a processor that executes the computer executable components stored in the memory. The computer executable components comprise an acquisition component that acquires data for a compound, the data defining a first mass spectrometry spectrum for the compound, an evaluation component that, based on the data, and employing a neural network that is trained on an input dataset comprising an acquisition metric, and employing an associated score that is associated with the acquisition metric, generates a recommendation to perform a mass spectrometry action for the compound, and an execution component that, based on the recommendation, directs execution of the mass spectrometry action at a mass spectrometer and obtaining a mass spectrum result.

In accordance with another embodiment, a computer-implemented method can comprise comparing, by a system operatively coupled to a processor, first data for a compound to an input data set comprising one or more acquisition metrics and one or more associated scores that are associated with the one or more acquisition metrics, for the compound, and based on the comparison, and on an obtained metric of interest associated with the compound, and employing a neural network trained on the input dataset, generating, by the system, a first recommendation to perform a recommended mass spectrometry action for the compound, wherein the recommended mass spectrometry action comprises use of a mass spectrometry device to obtain acquisition of the compound.

In accordance with still another embodiment, a computer program product facilitating a process for reinforcement learning-based mass spectrometry control, the computer program product comprising a computer readable storage medium having program instructions embodied therewith, and the program instructions executable by a processor to cause the processor to generate, by the processor, an input dataset, comprising one or more acquisition metrics and one or more associated scores that are associated with the one or more acquisition metrics, corresponding to a type of compound, wherein the one or more associated scores define probabilities that one or more thresholds corresponding to the one or more acquisition metrics will be satisfied by a mass spectrometry action performed for the type of compound, train, by the processor, a neural network on the input dataset, and generate, by the processor, a recommended mass spectrometry action for obtaining second data on a compound, of the type of compound, by employing the neural network to compare first data for the compound to the input dataset associated with the type of compound.

Other aspects of the invention are set out in the following numbered clauses:

1. A system, comprising:

a memory that stores computer executable components; and
a processor that executes the computer executable components stored in the memory, wherein the computer executable components comprise:

an acquisition component that acquires data for a compound, the data defining a first mass spectrometry spectrum for the compound;
an evaluation component that, based on the data, and employing a neural network that is trained on an input dataset comprising an acquisition metric, and employing an associated score that is associated with the acquisition metric, generates a recommendation to perform a mass spectrometry action for the compound; and
an execution component that, based on the recommendation, directs execution of the mass spectrometry action at a mass spectrometer and obtaining a mass spectrum result.

2. The system of clause 1, wherein the evaluation component further specifies an amount of a resource to employ for the mass spectrometry action.

3. The system of clause 1, wherein the evaluation component further specifies the compound, or a fragmented compound, from the first mass spectrometry spectrum, as a target of the mass spectrometry action.

4. The system of clause 1, wherein the evaluation component further specifies a plurality of targets corresponding to the compound, to be fragmented from the compound, or corresponding to one or more fragmented compounds from the first mass spectrometry spectrum, for which to obtain additional data by performance of the mass spectrometry action.

5. The system of clause 1, further comprising:
a metric component that obtains a metric of interest for the compound,
wherein the recommendation to perform the mass spectrometry action is at least partially based on the metric of interest.

6. The system of clause 1, further comprising:

a reward component that generates a reward indicator resulting from the recommendation of the mass spectrometry action, or from an execution of the mass spectrometry action recommended; and

an updating component that updates one or more weights employed by the NN or performs an adjustment to the acquisition metric, based on the reward indicator.

7. The system of clause 1, further comprising:
an updating component that updates the neural network according to a set of reward indicators amortized over time and obtained based on a plurality of recommendations of generations by the evaluation component, including the recommendation to perform the mass spectrometry action.

8. A computer-implemented method, comprising:

comparing, by a system operatively coupled to a processor, first data for a compound to an input data set comprising one or more acquisition metrics and one or more associated scores that are associated with the one or more acquisition metrics, for the compound; and

based on the comparison, and on an obtained metric of interest associated with the compound, and employing a neural network trained on the input dataset, generating, by the system, a first recommendation to perform a recommended mass spectrometry action for the compound, wherein the recommended mass spectrometry action comprises use of a mass spectrometry device to obtain acquisition of the compound.

9. The computer-implemented method of clause 8, further comprising:

identifying, by the system, the compound and a second compound from a mass spectrometry spectrum defined by the first data; and

generating in parallel, by the system, the fist recommendation and a second recommendation for the second compound.

10. The computer-implemented method of clause 8, wherein the first recommendation is based on historical data defining one or more results of acquisition of the compound caused by mass spectrometry analysis or other separation analysis of the compound.

11. The computer-implemented method of clause 8, further comprising:
generating, by the system, a dataset matrix for the compound, based on the input dataset, and comprising the one or more associated scores, wherein the one or more associated scores define probabilities that one or more thresholds corresponding to the one or more acquisition metrics will be satisfied by one or more additional mass spectrometry actions performed for the compound.

12. The computer-implemented method of clause 8, further comprising:
evaluating, by the system, the recommended mass spectrometry action, resulting in a reward indicator obtained by the system, wherein the reward indicator is employed to update one or more weights employed by the neural network.

13. The computer-implemented method of clause 8, further comprising:
generating, by the system, an associated score as a number between 0 and 1.

14. The computer-implemented method of clause 8, further comprising:
prior to the employing of the neural network to generate the recommended mass spectrometry action, correlating, by the system, the input dataset to the metric of interest.

15. The computer-implemented method of clause 8, further comprising:
selecting, by the system, a recommended mass spectrometry action comprising a fragmented acquisition that acquires the compound.

16. The computer-implemented method of clause 8, further comprising:
selecting, by the system, the recommended mass spectrometry action by the neural network from an action database of available mass spectrometry actions, available mass spectrometry actions are capable of being performed using one or more mass spectrometry devices communicatively coupled to the system.

17. A computer program product facilitating a process for reinforcement learning-based mass spectrometry control, the computer program product comprising a computer readable storage medium having program instructions embodied therewith, and the program instructions executable by a processor to cause the processor to:

generate, by the processor, an input dataset, comprising one or more acquisition metrics and one or more associated scores that are associated with the one or more acquisition metrics, corresponding to a type of compound,
wherein the one or more associated scores define probabilities that one or more thresholds corresponding to the one or more acquisition metrics will be satisfied by a mass spectrometry action performed for the type of compound;
train, by the processor, a neural network on the input dataset; and
generate, by the processor, a recommended mass spectrometry action for obtaining second data on a compound, of the type of compound, by employing the neural network to compare first data for the compound to the input dataset associated with the type of compound.

18. The computer program product of clause 15, wherein the generating of the recommended mass spectrometry action is further based on a metric of interest for the type of compound, which metric of interest is provided by a user entity to tailor functioning of the neural network.

19. The computer program product of clause 15, wherein the generating of the recommended mass spectrometry action further comprises specifying, by the processor, an amount of a resource to employ for the recommended mass spectrometry action, based on historical data defining acquisition of the compound caused by mass spectrometry analysis of the type of compound.

20. The computer program product of clause 15, wherein the recommended mass spectrometry action comprises a fragmented acquisition that is a mass spectrometry/mass spectrometry (MS2) acquisition for the compound or a mass spectrometry/-mass spectrometry/mass spectrometry (MS3) acquisition for the compound.

DETAILED DESCRIPTION

The following detailed description is merely illustrative and is not intended to limit embodiments and/or application or utilization of embodiments. Furthermore, there is no intention to be bound by any expressed or implied information presented in the preceding Summary section, or in the Detailed Description section. One or more embodiments are now described with reference to the drawings, wherein like reference numerals are utilized to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

Various operations can be described as multiple discrete actions or operations in turn, in a manner that is most helpful in understanding the subject matter disclosed herein. However, the order of description should not be construed as to imply that these operations are necessarily order dependent. In particular, these operations can be performed in an order different from the order of presentation. Operations described can be performed in a different order from the described embodiment. Various additional operations can be performed, and/or described operations can be omitted in additional embodiments.

Turning now to the subject of material analysis and the one or more embodiments described herein, when performing material analysis using mass spectrometry (e.g., mass spectrometry experiments), an ultimate goal can be to determine a maximum amount of compounds (also referred to herein as "precursors") within a given amount of time. This given amount of time can be defined as a requested period until results, such as where cost of time can be defined in terms of one or more of instrument depreciation, analyst cost and/or reagent cost for the time period.

In general, a mass spectrometer is device that can detect, identify and quantify molecules within samples based on molecular mass-to-charge ratio after ionization. That is, mass spectrometers can employ various ionization methods and can be used in tandem with different separation techniques.

Mass spectrometry (MS) experiments can be complicated endeavors employing a plurality of acquisition metrics to tune. Extensive work can go into optimizing methodologies and their acquisition metrics, which acquisition metrics can be complicated by changing sample, batch and experimental effects.

One existing approach, a data dependent acquisition (DDA) experiment, involves acquiring a MS1 spectrum, processing the MS1 spectrum, and proceeding to acquire MSn spectra for prospective compounds (wherein n is a total number of mass analysis stages, such as for MS2, MS3, etc.). In such cases, an isolation event can be a mass analysis stage. For further example, a DDA experiment can often employ MS2 acquisitions and a TMT-DDA experiment can employ an MS3 acquisition. The MSn spectra can be used for relative quantification when tandem mass tag (TMT) technology is employed, or only for identification purposes when a label-free (LF) strategy is used and where quantification is done with MS1 spectra, without being limited thereto.

Such LF-DDA can achieve good identification and quantification coverage, which can come at the cost of reproducibility, due to stochastic triggering of prospective compounds, and at the cost of sensitivity due to using MS1 for quantification. TMT-DDA may have a better throughput due to the sample multiplexing but has issues with reproducibility and coverage.

Another existing approach, a data independent acquisition (DIA) experiment, involves iteratively acquiring a set of MS/MS spectra that span a prospective precursor range. A DIA can, in instances, achieve a higher reproducibility and simplicity, at some cost to sensitivity and specificity due to spectral multiplexing.

As used herein, an "MS/MS" spectrum refers to a first mass analysis by way of an isolation process, a fragmentation event, and a final mass analysis generating a mass spectrum. It is noted that additional "/MS" can be added if this process is continued on to an isolation+fragmentation of first stage fragments (e.g., MS/MS/MS). It is noted that MS/MS = MS2 and MS/MS/MS = MS3.

A series of these acquisitions can span a precursor m/z space, which refers to a range of precursor mass-to-charge (m/z) values, i.e., a minimum and maximum value, which contain all of the precursor m/z values. For example, for a peptide analysis, a range of m/z 400 to 1000, or 400 to 1200, can contain a large fraction of the compounds in the sample, and [400,1000] could be selected as a precursor m/z space to be addressed by an experiment.

Yet another existing approach, a targeted MSn (tMSn) experiment, can have increased sensitivity over the other existing approaches, but the smallest number of compounds identified. Although under certain conditions like low sample amount and low total number of addressable compounds, a number of quantified compounds for a tMSn experiment can approach and/or exceed the other existing approaches.

These types of optimization can generally be incorporated into automated workflows. However, these types of optimizations are generally based on a user entity providing the acquisition metrics and MS settings to employ to allow for the achievement of a maximum amount of compounds acquired.

As used herein, "real time" can refer to performing one or more optimizations and/or changes to an experiment during operation of the one or more mass spectrometry devices (e.g., in real time).

That is the structure of the experiments and the gates and thresholds controlling the transition from one state to another is still very much explicitly defined by the programmer, which can have benefits in terms of predictability, but which can fail to fully exploit the instrument capabilities or to adapt to different experimental conditions. For example, the target number of ions/spectrum or the signal threshold controlling an MS to MS2 transition may vary depending on LC gradient length, sample type and/or the chemical details of a particular elution time. It can be the case that, in one or more specific parts of an experiment, there is extra time to devote to analysis of specific compounds, while at other times of the experiment, instrument resources should be more evenly spread among several eluting compounds, such as due to the specific details of compound elution from a chromatography system. These details are difficult or impossible to capture using existing optimization methods.

To account for one or more inabilities or deficiencies of existing frameworks (e.g., existing material analysis frameworks employing spectrum comparison), one or more embodiments are described herein that can specify high-level outcomes and employ a neural network (NN) trained based on a reinforcement learning-based (RL-based) approach to achieve one or more of the high-level outcomes. The RL-trained NN can automatically determine acquisition metrics and operation settings to recommend and/or direct subsequent acquisitions, and/or to define such acquisitions, based on the environment (e.g., the m/z precursor space) resulting from each subsequent and/or further fragmenting acquisition.

That is, it can be desired to control such experiments at a higher level than is enabled by existing approaches, by instead setting one or more metrics of interest of total acquisition time, number of compounds to acquire and/or types of compounds to acquire, as opposed to controlling experiments at a lower level of granulation involving each and every acquisition metric and MS operation setting.

Accordingly, one or more embodiments herein can allow for evolved compound-specific acquisition metrics without explicit programming of the acquisition metrics at each stage of an experiment (e.g., for each next acquisition, whether being a subsequent acquisition or a further fragmenting acquisition such as an MS/MS acquisition or MS/MS/MS acquisition).

Discussion next turns to reinforcement learning (RL), which is a field of optimization that can be employed for directing the actions of an agent (e.g., a system, device, processor, etc.) where the environment is changing and uncertain and/or where writing out explicit directives for the actions can be brittle and inflexible. Creating a program that allows a robot to stand up and run, or to clean a house are good examples of such situations, where it can be easy to explain what we want to happen, but difficult to write out explicit directions to achieve the goals under a broad range of circumstances.

Using conventional RL vocabulary, an agent interacts with an environment by performing actions. During training, the agent receives information about the environment state, selects an action which updates the environment, and receives a reward. The reward can be amortized over future states to help the system find actions that do not result in immediate rewards but pay off later. For example, in an extreme case, the agent can receive little or no reward (or penalty) until the end of a set of many steps, as may be the case for training an agent to navigate to a goal, or when an agent experiences something catastrophic (falling into a hole). This process can repeated over "episodes" comprising one or more MS experiments, such as liquid chromatography MS experiments. Typically, many hundreds or thousands of training episodes are needed to train an effective agent for a particular task.

In one or more embodiments described herein, using the above generic RL framework, there can be an opportunity to employ in silico optimization concepts to utilize frameworks of reinforcement learning to not only optimize acquisition metrics and/or MS operation settings, but also to direct operation of one or more mass spectrometry device in real time. For example, a general optimization scheme can comprise determining a list of experiments with their respective different acquisition metrics and MS operation settings, employing an RL-trained NN, and performing the experiments either in real-life or in simulation, resulting in respective corresponding experiment scores. The experiment scores can take a form of a number of unique identifications metrics, referring to a number of compounds that were detected in the experiment and the quality of those detections (ex. their signal-to-noise ration S/N). The optimization could be a simple enumeration over a grid of experimental acquisition metrics, or a more advanced optimization, using a design of experiments, genetic algorithm, or particle optimization for example.

Discussion next turns to a general discussion of one or more scientific instrument systems disclosed herein, as well as related methods, computing devices, and computer-readable media. For example, in one or more embodiments, a system can comprise a memory that stores computer executable components and a processor that executes the computer executable components stored in the memory. The computer executable components comprise an acquisition component that acquires data for a compound, the data defining a first mass spectrometry spectrum for the compound, an evaluation component that, based on the data, employing a neural network that is trained on an input dataset comprising one or more acquisition metrics and employing one or more associated scores that are associated with the one or more acquisition metrics, generates a recommendation to perform a mass spectrometry action for the compound, and an execution component that, based on the recommendation, directs execution of the mass spectrometry action at a mass spectrometer and obtains a mass spectrum result.

The one or more embodiments disclosed herein can achieve improved performance relative to existing approaches. For example, based on RL training and on historical data on which a NN can be RL trained, a system employing an RL-trained NN, as described herein, can employ better compound-specific acquisition actions than can be merely pre-programmed by an administrative entity. That is, such system can learn to recognize when resources should be spent during current acquisitions and during future recommended or future directed acquisition actions. As noted above, there can be precursor environments where expanded accumulation times can result in lower limits of detection, or there can be precursor environments where rapid acquisition time can be necessary in view of the large number of compounds entering the mass spectrometer.

Moreover, an embodiment described herein can beneficially provide focus/direction for plural compounds at once, even based on different recommended acquisition actions. That is, an embodiment described herein can employ mixed-optimization techniques to achieve the best results based on the selected high-level goals, such as specificity of analysis, increased probability of accuracy and/or increased compound detection numbers.

Further, the embodiments described herein can be adapted to different types of experiments, labeling technologies and/or mass or cross section analyzing technologies. That is, depending on a level of sample description or applicable high-level experiment goals, a framework described herein can be adapted to fit the sample. For example, rewards, high level goals, available acquisition actions, etc. can be inputted into the system and into the NN, with the NN being updated to operate within confines of the inputs.

The embodiments disclosed herein thus provide improvements to scientific instrument technology (e.g., improvements in the computer technology supporting such scientific instruments, among other improvements).

Various ones of the embodiments disclosed herein can improve upon existing approaches to achieve the technical advantages of high accuracy and/or high quantity of identifications of material compounds, through the use of a reinforcement learning agent (e.g., a NN trained using RL). That is, generation of output data can be achieved without specific lower-level control of acquisition metrics and/or operation settings for each and every step of a multi-step material analysis experiment using MS.

Such technical advantages are not achievable by routine and existing approaches, and all user entities of systems including such embodiments can benefit from these advantages (e.g., by assisting the user entity in the performance of a technical task, such as identification of one or more compounds, by means of a guided human-machine-NN interaction process).

The technical features of the embodiments disclosed herein (e.g., the ability to generate one or more MS results using RL-based control) are thus decidedly unconventional in the field of material analysis, as are combinations of the features of the embodiments disclosed herein.

As discussed further herein, various aspects of the embodiments disclosed herein can improve the functionality of a computer itself. Additionally, the computational and user interface features disclosed herein do not involve only the collection and comparison of information but instead apply new analytical and technical techniques to change the operation of the computer-analysis of material compounds. For example, based on the results outputs of an experiment, and based on one or more reward indicators corresponding to the results outputs, an updating process can be employed by the system/NN combination to reinforce good recommendations by the NN. Accordingly, a non-limiting system described herein, comprising a material analysis system and a NN, can be self-improving.

The present disclosure thus introduces functionality that neither an existing computing device, nor a human, could perform. Rather, such existing computing devices would instead require low level control of MS-based or MS-corresponding experiments, such as including, but not limited to, specific control of acquisition metrics and operation settings for each stage of a multi-stage experiment. In view of the time, energy and human error involved, in addition to the lack of computerized referencing using historical data, it is not practical to operate within the confines of existing approaches.

Accordingly, the embodiments of the present disclosure can serve any of a number of technical purposes, such as controlling a specific technical system or process; determining from measurements how to control a machine; digital audio, image, or video enhancement or analysis; separation of material sources in a mixed signal; generating data for reliable and/or efficient transmission or storage; providing estimates and confidence intervals for material samples; or providing a faster processing of sensor data. In particular, the present disclosure provides technical solutions to technical problems, including, but not limited to, providing acquisition recommendations and/or directing acquisition, using historical data to make acquisition recommendations, generating updates scoring corresponding to one or more probabilities; and/or providing a faster, more thorough and/or more efficient processing of material compounds.

The embodiments disclosed herein thus provide improvements to material analysis technology (e.g., improvements in the computer technology supporting material analysis, among other improvements).

As used herein, the phrase "based on" should be understood to mean "based at least in part on," unless otherwise specified.

As used herein, the term "component" can refer to an atomic element, molecular element, phase of an atomic or molecular element, or combination thereof.

As used herein, the terms "compound" and "precursor" can be used interchangeably.

As used herein, the term "data" can comprise metadata.

As used herein, the terms "entity," "requesting entity," and "user entity" can refer to a machine, device, component, hardware, software, smart device, party, organization, individual and/or human.

One or more embodiments are now described with reference to the drawings, where like referenced numerals are used to refer to like drawing elements throughout. In the following description, for purposes of explanation, numerous specific details are set forth in order to provide a more thorough understanding of the one or more embodiments. It is evident in various cases, however, that the one or more embodiments can be practiced without these specific details.

Further, it should be appreciated that the embodiments depicted in one or more figures described herein are for illustration only, and as such, the architecture of embodiments is not limited to the systems, devices and/or components depicted therein, nor to any particular order, connection and/or coupling of systems, devices and/or components depicted therein.

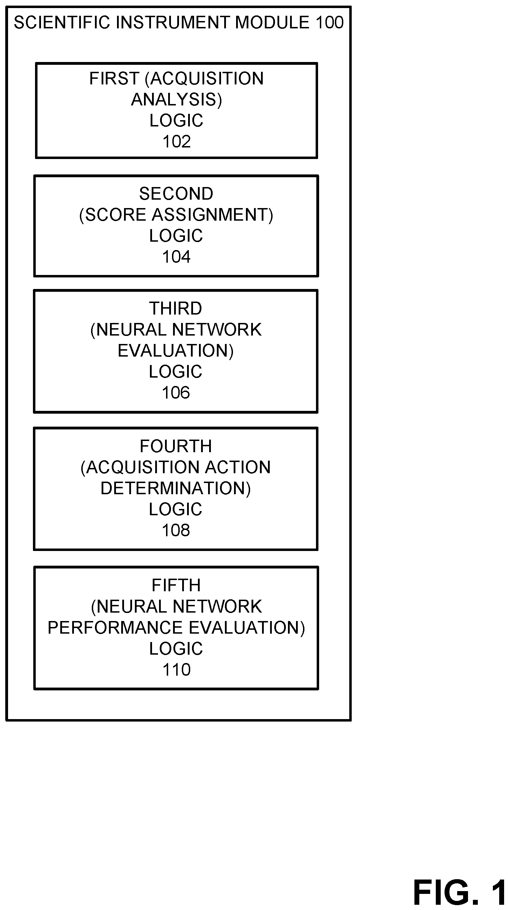

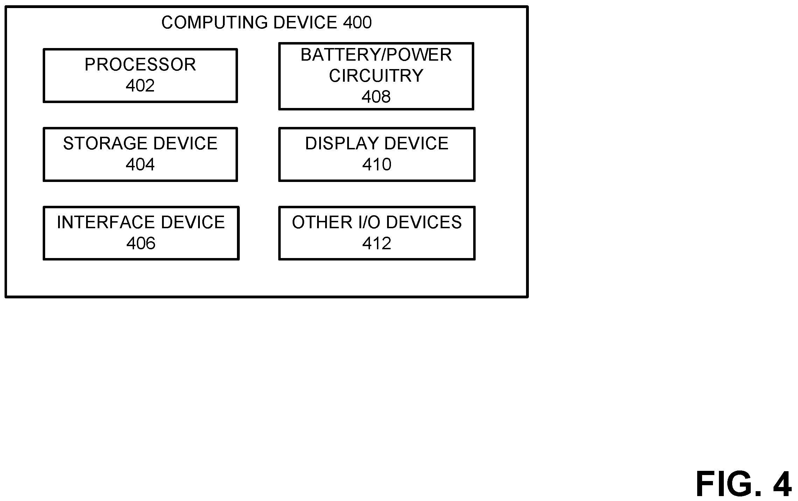

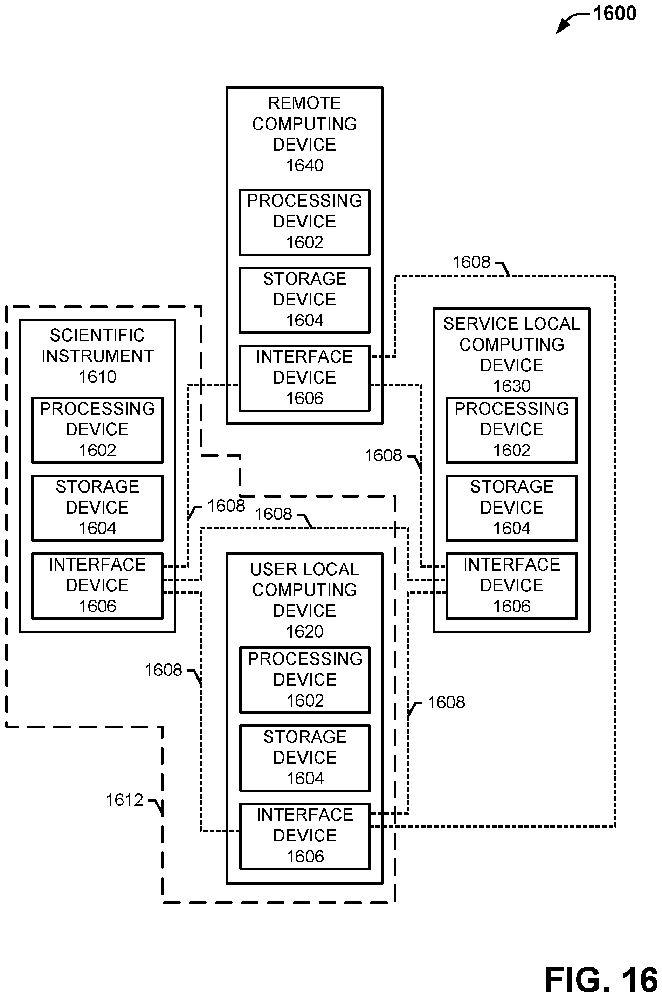

Turning now in particular to the one or more figures, and first to FIG. 1, illustrated is a block diagram of a scientific instrument module 100 for performing material analysis operations using an RL-trained NN and for performing at least one or more aspects of the training, in accordance with various embodiments described herein. The scientific instrument module 100 can be implemented by circuitry (e.g., including electrical and/or optical components), such as a programmed computing device. The logic of the scientific instrument module 100 can be included in a single computing device or can be distributed across multiple computing devices that are in communication with each other as appropriate. Examples of computing devices that can, singly or in combination, implement the scientific instrument module 100 are discussed herein with reference to the computing device 400 of FIG. 4, and examples of systems of interconnected computing devices, in which the scientific instrument module 100 can be implemented across one or more of the computing devices, is discussed herein with reference to the scientific instrument system 1600 of FIG. 16.

The scientific instrument module 100 can include first logic 102, second logic 104, third logic 106, fourth logic 108 and fifth logic 110. As used herein, the term "logic" can include an apparatus that is to perform a set of operations associated with the logic. For example, any of the logic elements included in the module 100 can be implemented by one or more computing devices programmed with instructions to cause one or more processing devices of the computing devices to perform the associated set of operations. In a particular embodiment, a logic element can include one or more non-transitory computer-readable media having instructions thereon that, when executed by one or more processing devices of one or more computing devices, cause the one or more computing devices to perform the associated set of operations. As used herein, the term "module" can refer to a collection of one or more logic elements that, together, perform a function associated with the module. Different ones of the logic elements in a module can take the same form or can take different forms. For example, some logic in a module can be implemented by a programmed general-purpose processing device, while other logic in a module can be implemented by an application-specific integrated circuit (ASIC). In another example, different ones of the logic elements in a module can be associated with different sets of instructions executed by one or more processing devices. A module can omit one or more of the logic elements depicted in the associated drawing; for example, a module can include a subset of the logic elements depicted in the associated drawing when that module is to perform a subset of the operations discussed herein with reference to that module.

The first logic 102 can compare input data for a compound with acquisition metrics, historical data and/or compound-type data to generate a dataset matrix comprising scoring defining probabilities of achieving one or more acquisition metrics and/or metrics of interest. That is, the first logic 102 can generate and/or transform data for use in performing a recommendation of an acquisition action.

The second logic 104 can generate one or more scores for the aforementioned scoring. That is, the second logic 104 can aid in defining (e.g., including ranking) acquisition metrics and/or metrics of interest on which to focus, and on which a recommended acquisition action can be based.

The third logic 106 can comprise performance of evaluation by an RL-trained neural network, such as based on any of the outputs of the first logic 102 or the second logic 104.

The fourth logic 108 can comprise output of a recommended acquisition action or output of a direction to execute an acquisition action.

The fifth logic 110 can comprise evaluation of performance of the NN, based on the output of the fourth logic 108. That is, the fifth logic 110 can employ a reward system to reinforce outputs that meet and/or satisfy one or more acquisition metrics and/or metrics of interest.

As used herein, an acquisition metric can be detection probability, signal intensity, fit to predicted isotopic cluster, mass accuracy, delta time between estimated current time and expected elution time and/or whether acquisition was acquired for a same precursor on a last iteration (e.g., step or stage) of an experiment, without being limited thereto. Another acquisition metric can be identification of a neighbor compound, where identification of one compound corresponds to an increase in a probability that another compound, such as a compound of interest, is present or soon will be present.

Differently, as used herein, a metric of interest can be a high-level aspect, such as quantity of precursors identified, specificity of precursors identified, precision of precursor abundance measurements, and/or speed of precursor identification, without being limited thereto.

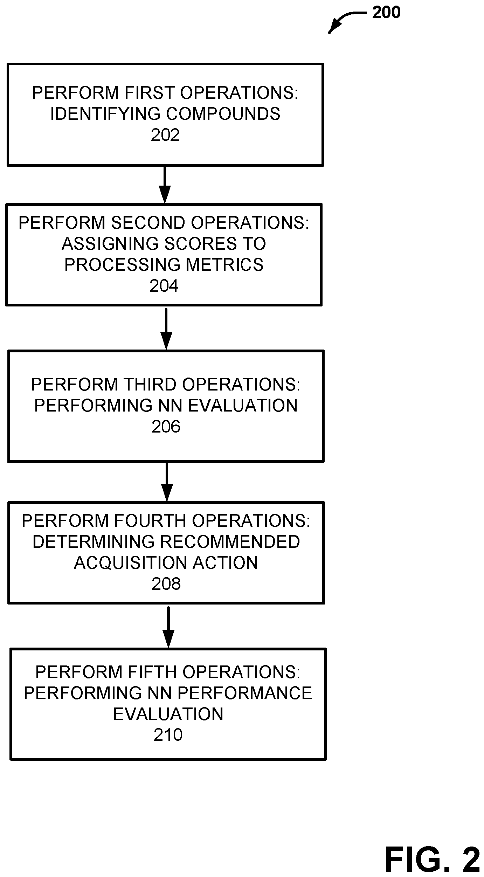

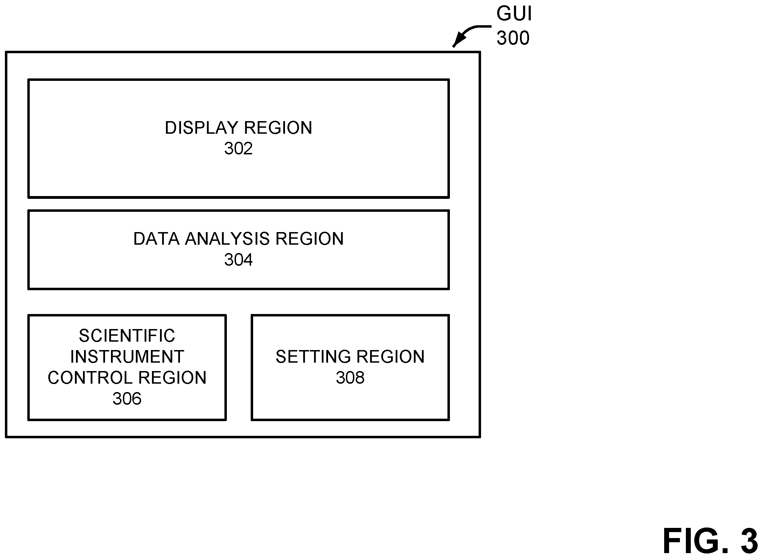

FIG. 2 illustrates a flow diagram of a method 200 of performing operations, by the scientific instrument module 100, in accordance with various embodiments. Although the operations of the method 200 can be illustrated with reference to particular embodiments disclosed herein (e.g., the scientific instrument module 100 discussed herein with reference to FIG. 1, the GUI 300 discussed herein with reference to FIG. 3, the computing device 400 discussed herein with reference to FIG. 4, and/or the scientific instrument system 1600 discussed herein with reference to FIG. 16), the method 200 can be used in any suitable setting to perform any suitable operations. Operations are illustrated once each and in a particular order in FIG. 2, but the operations can be reordered and/or repeated as desired and appropriate (e.g., different operations performed can be performed in parallel, as suitable).

At 202, first operations can be performed. For example, the first logic 102 of the module 100 can perform the first operations 202. The first operations 202 can include identifying one or more compounds with which to employ for an experiment (e.g., from which to identify one or more precursors).

As used herein, a precursor is a compound, and the term can be employed to refer to a compound that is identified from another compound.

At 204, second operations can be performed. For example, the second logic 104 of the module 100 can perform the second operations 204. The second operations 204 can include assigning scores to processing parameters and/or to metrics of interest.

At 206, third operations can be performed. For example, the third logic 106 of the module 100 can perform the third operations 206. The third operations 206 can include performing evaluation of data using an RL-trained neural network.

At 208, fourth operations can be performed. For example, the fourth logic 108 of the module 100 can perform the fourth operations 208. The fourth operations 208 can include determining one or more recommended acquisition actions and/or directing execution of one or more acquisition actions.

At 210, fifth operations can be performed. For example, the fifth logic 110 of the module 100 can perform the fifth operations 210. The fifth operations 210 can include performing a performance evaluation of the NN based on the output from the fourth operations. The fifth operations 210 can include employing a reward system and updating data and/or function of the NN based on the reward system.

The scientific instrument methods disclosed herein can include interactions with a user entity (e.g., via the user local computing device 1620 discussed herein with reference to FIG. 16). These interactions can include providing information to the user entity (e.g., information regarding the operation of a scientific instrument such as the scientific instrument 1610 of FIG. 16, information regarding a sample being analyzed or other test or measurement performed by a scientific instrument, information retrieved from a local or remote database, or other information) or providing an option for a user entity to input commands (e.g., to control the operation of a scientific instrument such as the scientific instrument 1610 of FIG. 16, or to control the analysis of data generated by a scientific instrument), queries (e.g., to a local or remote database), or other information. In some embodiments, these interactions can be performed through a graphical user interface (GUI) that includes a visual display on a display device (e.g., the display device 410 discussed herein with reference to FIG. 4) that provides outputs to the user entity and/or prompts the user entity to provide inputs (e.g., via one or more input devices, such as a keyboard, mouse, trackpad, or touchscreen, included in the other I/O devices 412 discussed herein with reference to FIG. 4). The scientific instrument system 1600 disclosed herein can include any suitable GUIs for interaction with a user entity.

Turning next to FIG. 3, depicted is an example GUI 300 that can be used in the performance of some or all of the methods described herein, in accordance with various embodiments described herein. As noted above, the GUI 300 can be provided on a display device (e.g., the display device 410 discussed herein with reference to FIG. 4) of a computing device (e.g., the computing device 400 discussed herein with reference to FIG. 4) of a scientific instrument system (e.g., the scientific instrument system 1600 discussed herein with reference to FIG. 16), and a user entity can interact with the GUI 300 using any suitable input device (e.g., any of the input devices included in the other I/O devices 412 discussed herein with reference to FIG. 4) and input technique (e.g., movement of a cursor, motion capture, facial recognition, gesture detection, voice recognition, actuation of buttons, etc.).

The GUI 300 can include a data display region 302, a data analysis region 304, a scientific instrument control region 306, and a settings region 308. The particular number and arrangement of regions depicted in FIG. 3 is merely illustrative, and any number and arrangement of regions, including any desired features thereof, can be included in a GUI 300.

The data display region 302 can display data generated by a scientific instrument (e.g., the scientific instrument 1610 discussed herein with reference to FIG. 16). For example, the data display region 302 can display one or more output results 682 which can comprise text, graphs, charts, matrices and/or spectra, without being limited thereto.

The data analysis region 304 can display the results of data analysis (e.g., the results of analyzing the data illustrated in the data display region 302 and/or other data). For example, the data analysis region 304 can display one or more of the compound results 682 and/or one or more reward indicators 722. In one or more cases, the data analysis region 304 can display a list, flow chart or other schematic of acquisition actions taken and/or recommended relative to an experiment. In one or more embodiments, the data display region 302 and the data analysis region 304 can be combined in the GUI 300 (e.g., to include data output from a scientific instrument, and some analysis of the data, in a common graph or region).

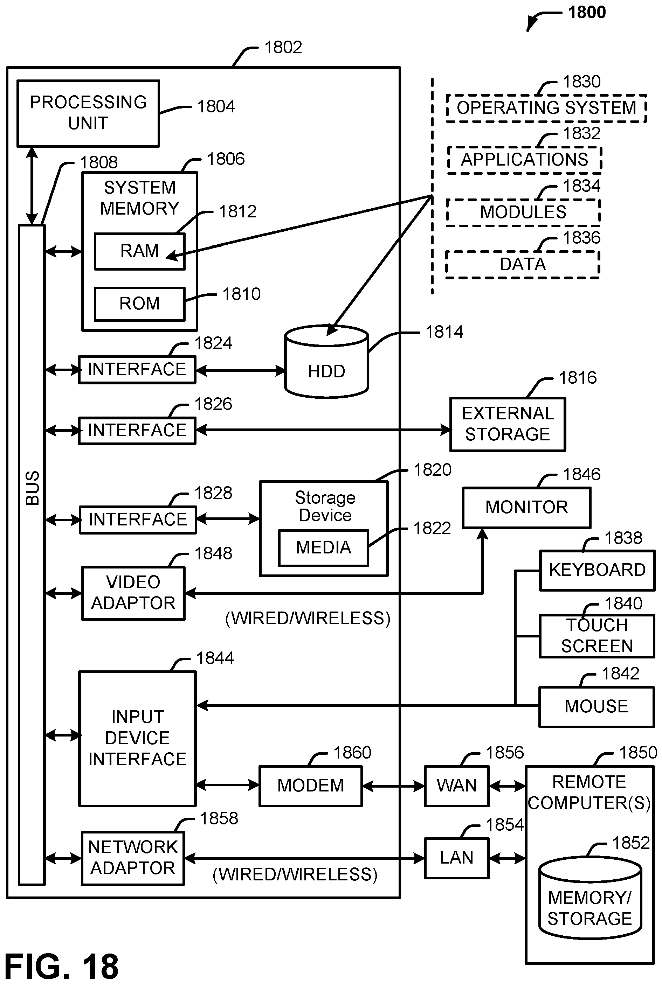

The scientific instrument control region 306 can include options that allow the user entity to control a scientific instrument (e.g., the scientific instrument 1810 discussed herein with reference to FIG. 18). For example, the scientific instrument control region 306 can include one or more controls for inputting one or more metrics of interest.

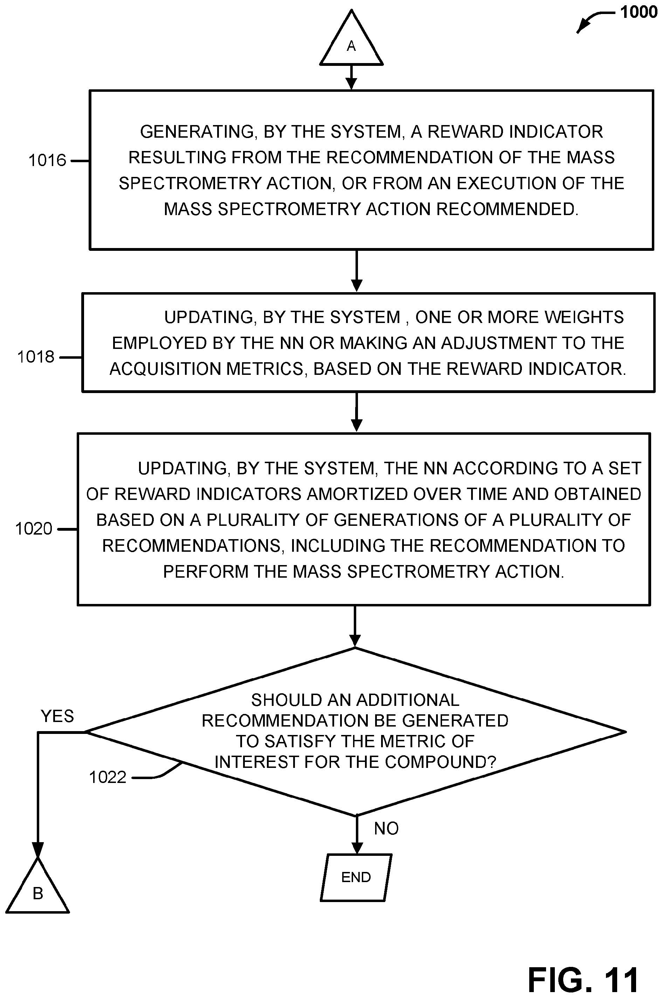

The settings region 308 can include options that allow the user entity to control the features and functions of the GUI 300 (and/or other GUIs) and/or perform common computing operations with respect to the data display region 302 and data analysis region 304 (e.g., saving data on a storage device, such as the storage device 404 discussed herein with reference to FIG. 4, sending data to another user entity, labeling data, etc.). For example, the settings region 308 can include one or more options to alter color, fill or format of illustrations, such as illustrations 1150 and 1160 of FIG. 11.

As noted above, the scientific instrument module 100 can be implemented by one or more computing devices. Accordingly, discussion next turns to FIG. 4, which illustrates a block diagram of a computing device 400 that can perform some or all of the scientific instrument methods disclosed herein, in accordance with various embodiments. In one or more embodiments, the scientific instrument module 100 can be implemented by a single computing device 400 or by multiple computing devices 400. Further, as discussed below, a computing device 400 (or multiple computing devices 400) that implements the scientific instrument module 100 can be part of one or more of the scientific instrument 1610, the user local computing device 1620, the service local computing device 1630, or the remote computing device 1640 of FIG. 16.

The computing device 400 of FIG. 4 is illustrated as having a number of components, but any one or more of these components can be omitted or duplicated, as suitable for the application and setting. As illustrated, these components can include one or more of a processor 402, storage device 404, interface device 406, battery/power circuitry 408, display device 410 and other input/output (I/O) devices 412, as will be described below.

In one or more embodiments, one or more of the components included in the computing device 400 can be attached to one or more motherboards and enclosed in a housing (e.g., including plastic, metal, and/or other materials). In one or more embodiments, some these components can be fabricated onto a single system-on-a-chip (SoC) (e.g., an SoC can include one or more processors 402 and one or more storage devices 404). Additionally, in one or more embodiments, the computing device 400 can omit one or more of the components illustrated in FIG. 4. In one or more embodiments, the computing device 400 can include interface circuitry (not shown) for coupling to the one or more components using any suitable interface (e.g., a Universal Serial Bus (USB) interface, a High-Definition Multimedia Interface (HDMI) interface, a Controller Area Network (CAN) interface, a Serial Peripheral interface (SPI) interface, an Ethernet interface, a wireless interface, or any other appropriate interface). For example, the computing device 400 can omit a display device 410, but can include display device interface circuitry (e.g., a connector and driver circuitry) to which a display device 410 can be coupled.

The computing device 400 can include the processor 402 (e.g., one or more processing devices). As used herein, the term "processing device" can refer to any device or portion of a device that processes electronic data from registers and/or memory to transform that electronic data into other electronic data that can be stored in registers and/or memory. The processor 402 can include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 400 can include a storage device 404 (e.g., one or more storage devices). The storage device 404 can include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In one or more embodiments, the storage device 404 can include memory that shares a die with a processor 402. In such an embodiment, the memory can be used as cache memory and can include embedded dynamic random-access memory (eDRAM) or spin transfer torque magnetic random-access memory (STT-MRAM), for example. In one or more embodiments, the storage device 404 can include non-transitory computer readable media having instructions thereon that, when executed by one or more processing devices (e.g., the processor 402), cause the computing device 400 to perform any appropriate ones of or portions of the methods disclosed herein.

The computing device 400 can include an interface device 406 (e.g., one or more interface devices 406). The interface device 406 can include one or more communication chips, connectors, and/or other hardware and software to govern communications between the computing device 400 and other computing devices. For example, the interface device 406 can include circuitry for managing wireless communications for the transfer of data to and from the computing device 400. The term "wireless" and its derivatives can be used to describe circuits, devices, systems, methods, techniques, communications channels, etc., that can communicate data through the use of modulated electromagnetic radiation through a nonsolid medium. The term does not imply that the associated devices do not contain any wires, although in one or more embodiments the associated devices might not contain any wires. Circuitry included in the interface device 406 for managing wireless communications can implement any of a number of wireless standards or protocols, including but not limited to Institute for Electrical and Electronic Engineers (IEEE) standards including Wi-Fi (IEEE 802.11 family), IEEE 802.16 standards (e.g., IEEE 802.16-2005 Amendment), Long-Term Evolution (LTE) project along with any amendments, updates, and/or revisions (e.g., advanced LTE project, ultra mobile broadband (UMB) project (also referred to as "3GPP2"), etc.). In one or more embodiments, circuitry included in the interface device 406 for managing wireless communications can operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In one or more embodiments, circuitry included in the interface device 406 for managing wireless communications can operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In one or more embodiments, circuitry included in the interface device 406 for managing wireless communications can operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In one or more embodiments, the interface device 406 can include one or more antennas (e.g., one or more antenna arrays) to receipt and/or transmission of wireless communications.

In one or more embodiments, the interface device 406 can include circuitry for managing wired communications, such as electrical, optical, or any other suitable communication protocols. For example, the interface device 406 can include circuitry to support communications in accordance with Ethernet technologies. In one or more embodiments, the interface device 406 can support both wireless and wired communication, and/or can support multiple wired communication protocols and/or multiple wireless communication protocols. For example, a first set of circuitry of the interface device 406 can be dedicated to shorter-range wireless communications such as Wi-Fi or Bluetooth, and a second set of circuitry of the interface device 406 can be dedicated to longer-range wireless communications such as global positioning system (GPS), EDGE, GPRS, CDMA, Wi-MAX, LTE, EV-DO, or others. In one or more embodiments, a first set of circuitry of the interface device 406 can be dedicated to wireless communications, and a second set of circuitry of the interface device 406 can be dedicated to wired communications.

The computing device 400 can include battery/power circuitry 408. The battery/power circuitry 408 can include one or more energy storage devices (e.g., batteries or capacitors) and/or circuitry for coupling components of the computing device 400 to an energy source separate from the computing device 400 (e.g., AC line power).

The computing device 400 can include a display device 410 (e.g., multiple display devices). The display device 410 can include any visual indicators, such as a heads-up display, a computer monitor, a projector, a touchscreen display, a liquid crystal display (LCD), a light-emitting diode display, or a flat panel display.

The computing device 400 can include other input/output (I/O) devices 412. The other I/O devices 412 can include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 400, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 400 can have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer, etc.), a desktop computing device, or a server computing device or other networked computing component.

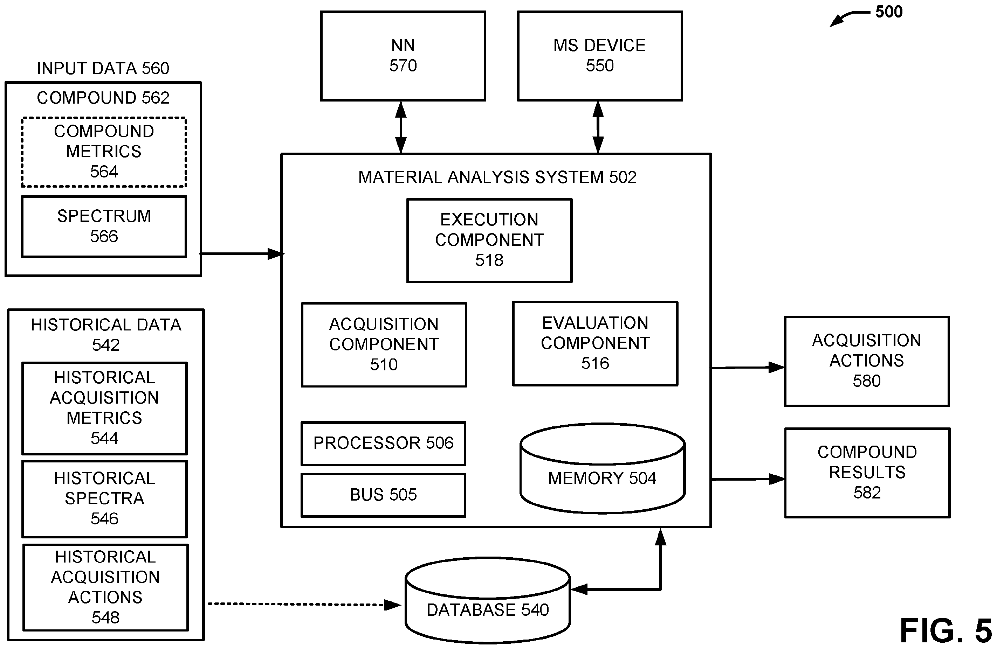

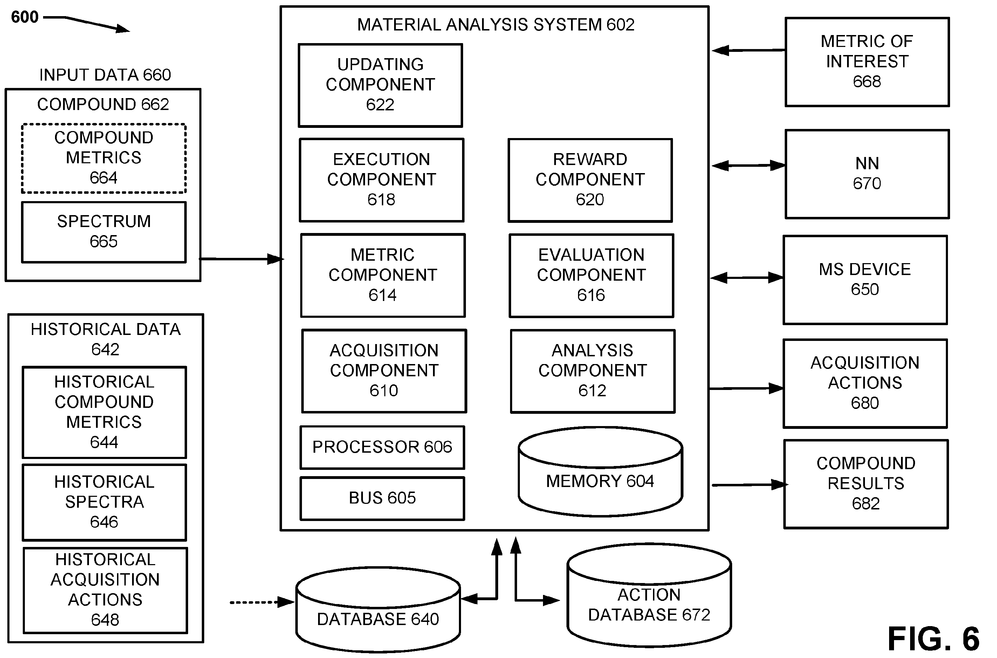

Referring next to FIGS. 5 and 6, in one or more embodiments, the non-limiting systems 500 and/or 600 illustrated at FIGS. 5 and 6, and/or systems thereof, can further comprise one or more computer and/or computing-based elements described herein with reference to a computing environment, such as the computing environment 1800 illustrated at FIG. 18. In one or more described embodiments, computer and/or computing-based elements can be used in connection with implementing one or more of the systems, devices, components and/or computer-implemented operations shown and/or described in connection with FIGS. 5 and/or 6 and/or with other figures described herein.

Turning first to FIG. 5, the figure illustrates a block diagram of an example, non-limiting system 500 that can comprise a material analysis system 502, a neural network (NN) 570 and an MS device 550. The material analysis system 502 can facilitate a process to employ an RL-trained NN to control an MS-based experiment.

In one or more embodiments, the material analysis system 502 can be at least partially comprised by the computing device 400.

In one or more embodiments, the material analysis system 502 can at least partially comprise the NN 570 and/or the MS device 550.

It is noted that the material analysis system 502 is only briefly detailed to provide but a lead-in to a more complex and/or more expansive material analysis system 602 as illustrated at FIG. 6. That is, further detail regarding processes that can be performed by one or more embodiments described herein will be provided below relative to the non-limiting system 600 of FIG. 6.

Still referring to FIG. 5, the material analysis system 502 can comprise at least a memory 504, bus 505, processor 506, acquisition component 510, evaluation component 516 and execution component 518. The processor 506 can be the same as the processor 402, comprised by the processor 402 or different therefrom. The memory 504 can be the same as the storage device 404, comprised by the storage device 404 or different therefrom.

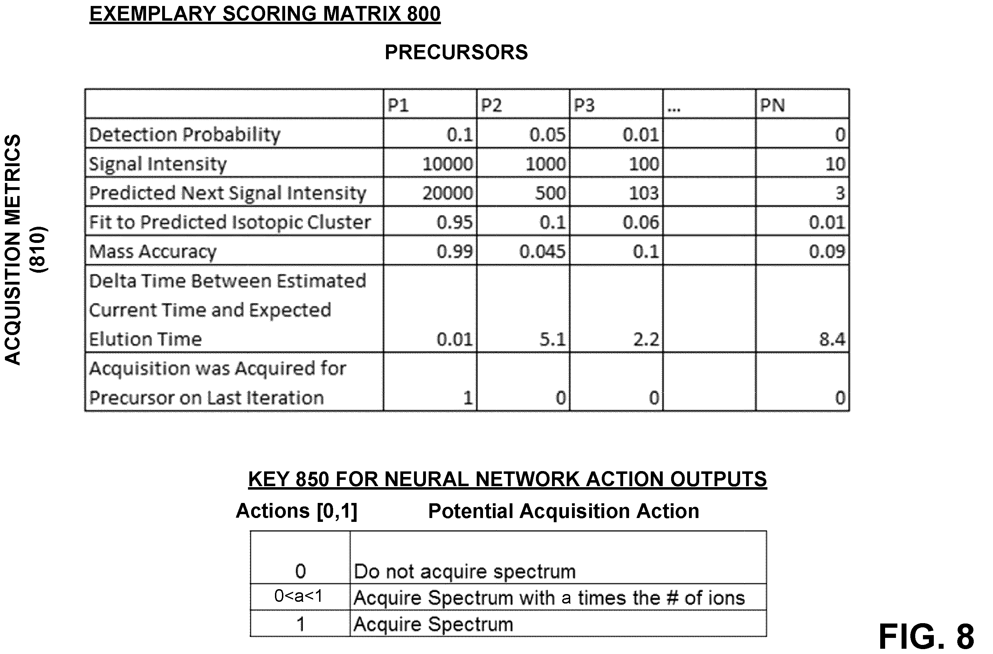

Using the above-noted components, the material analysis system 502 can employ an RL-trained NN to control an MS-based experiment to provide output information related to a compound 562 for being analyzed. That is, the material analysis system 502 can determine acquisition metrics 810 (FIG. 8) and/or operation settings for one or more stages of an MS experiment based on acquired data (e.g., input data 560), historical data 542 and/or analysis thereof. Based on these acquisition metrics 810, operation settings and/or historical data 542, the material analysis system can define at least one recommended acquisition action to take to achieve a metric of interest corresponding to the compound 562.

The metric of interest can be provided by a user entity and/or can be programmed into an experiment program being employed by the material analysis system 502. A metric of interest can comprise a focus on one or more of number of compounds identified, speed of identification, accuracy of identification and/or precision of identification without being limited thereto.

Generally, the acquisition component 510 can acquire data (e.g., input data 560; also referred to herein as first data) for a compound 562. In one or more embodiments, the data can define a first mass spectrometry spectrum 566 for the compound 562. The data can comprise one or more compound parameters 564 (e.g., physical phase temperature ranges, physical properties, mechanical properties, chemical properties and/or the like, without being limited thereto).

Based on the data and employing a neural network 570 that is trained on an input dataset comprising one or more acquisition metrics and one or more associated scores that are associated with the one or more acquisition metrics, the evaluation component 516 can generate a recommendation to perform a mass spectrometry action (e.g., acquisition action 580) for the compound 562. It is noted that the input dataset is different from the input data 560, but that the input data 560 can be employed to fill in, generate, update and/or transform the input dataset. The input dataset, such as an exemplary scoring matrix 800, will be described in greater detail below, relative to the material analysis system 602.

Based on the recommendation to perform a mass spectrometry action (e.g., acquisition action 580), the execution component 518 can direct execution of the mass spectrometry action at a mass spectrometer (e.g., MS device 550) and can obtain a mass spectrum result (e.g., comprising a compound result 582) therefrom.

The acquisition component 510, evaluation component 516 and execution component 518 can be operatively coupled to the processor 506 which can be operatively coupled to the memory 504. The bus 505 can provide for the operative coupling. The processor 506 can facilitate execution of the acquisition component 510, evaluation component 516 and execution component 518. The acquisition component 510, evaluation component 516 and execution component 518 can be stored at the memory 504.

In general, the non-limiting system 500 can employ any suitable method of communication (e.g., electronic, communicative, internet, infrared, fiber, etc.) to provide communication between the material analysis system 502, a database 540, the NN 570 the MS device 550 and/or any device associated with a user entity.

Turning next to FIG. 6, a non-limiting system 600 is illustrated that can comprise a material analysis system 602, NN 670, database 640 and MS device 550. Repetitive description of like elements and/or processes employed in respective embodiments is omitted for sake of brevity. Description relative to an embodiment of FIG. 5 can be applicable to an embodiment of FIG. 6. Likewise, description relative to an embodiment of FIG. 6 can be applicable to an embodiment of FIG. 5.

Generally, the material analysis system 502 can facilitate a process to employ an RL-trained NN to control an MS-based experiment.

In one or more embodiments, the material analysis system 602 can be at least partially comprised by the computing device 400.

In one or more embodiments, the material analysis system 602 can at least partially comprise the NN 670, the database 640 and/or the MS device 650.

The NN 670 can be and/or can comprise any suitable artificial intelligence aspect, neurons, layers of neurons, clusters of neurons and/or the like. The NN 670 can be initially trained using reinforcement learning by the updating component 622 and/or by any external training system. For example, during training of the NN 670, the NN 670 can receive information about an environment state, be requested to select an action which updates the environment, and receive a reward. The reward may be amortized over future states to help the NN 670 find actions that do not result in immediate rewards but pay off later. For example, in an extreme case, the NN 670 can receive little or no reward (or penalty) until the end of a set of many steps, as may be the case for training an agent to navigate to a goal, or when a NN 670 experiences something catastrophic (falling into a hole). This process is repeated over "episodes" comprising one or more LC-MS experiments. Typically, many hundreds or thousands of training episodes can be employed to train the NN 670 for material analysis of a particular compound or type of compound. As used herein, a "type" can comprise various compounds such as human tissue, mining gases, etc.

As used herein, an example of the NN 670 experiencing a catastrophic event can refer to use of a metric to characterize entire elution peaks (e.g., acquire 8-10 points per LC peak). The NN can have difficulty determining completion until an entire peak is characterized, thus resulting in beginning or intermediate actions relative to the peak resulting in low rewards. Rather, a reward amortization scheme can be employed over time, such as to transfer a final reward backwards to previous actions.

In one or more embodiments, an array of two or more NN's 670 can be trained and employed, such as for different precursors. In one or more examples, such two or more NN's 670 can be aggregated (e.g., stacked) to form a super NN that can be used to more rapidly and more efficiently produce recommended acquisition actions 720, such as relative to different precursors.

The database 640 can comprise historical data 542 in any suitable format, including but not limited to numbers, text, code, data, metadata, images, etc. The historical data 542 can comprise historical acquisition metrics 544, historical spectra 546 and/or historical acquisition actions 548. The historical acquisition metrics 544 can comprise ion quantity necessary for analysis, analysis temperature and/or analysis degradation parameters, without being limited thereto. The historical acquisition actions 548 can be those taken to achieve certain metrics of interest in past MS-based experiments, or in previous acquisitions in the same experiment.

The database 640 can be at least partially comprised by the material analysis system 602 in one or more embodiments.

The MS device 650 can be any suitable MS device that can provide material analysis output, such as one or more spectra.

One or more communications between one or more components of the non-limiting system 600 can be provided by wired and/or wireless means including, but not limited to, employing a cellular network, a wide area network (WAN) (e.g., the Internet), and/or a local area network (LAN). Suitable wired or wireless technologies for supporting the communications can include, without being limited to, wireless fidelity (Wi-Fi), global system for mobile communications (GSM), universal mobile telecommunications system (UMTS), worldwide interoperability for microwave access (WiMAX), enhanced general packet radio service (enhanced GPRS), third generation partnership project (3GPP) long term evolution (LTE), third generation partnership project 2 (3GPP2) ultra-mobile broadband (UMB), high speed packet access (HSPA), Zigbee and other 802.XX wireless technologies and/or legacy telecommunication technologies, BLUETOOTH®, Session Initiation Protocol (SIP), ZIGBEE®, RF4CE protocol, WirelessHART protocol, 6LoWPAN (Ipv6 over Low power Wireless Area Networks), Z-Wave, an advanced and/or adaptive network technology (ANT), an ultra-wideband (UWB) standard protocol and/or other proprietary and/or non-proprietary communication protocols.

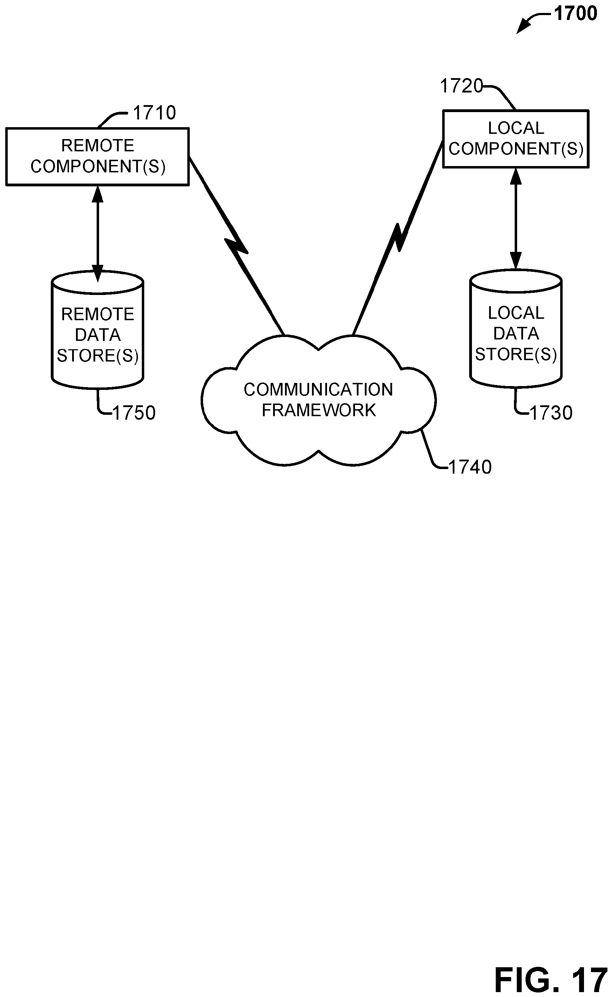

The material analysis system 602 can be associated with, such as accessible via, a cloud computing environment, such as the cloud computing environment 1700 of FIG. 17.

The material analysis system 602 can comprise a plurality of components. The components can comprise a memory 604, processor 606, bus 605, acquisition component 610, analysis component 612, metric component 614, evaluation component 616, execution component 618, reward component 620 and/or updating component 622. Using these components, the material analysis system 602 can output one or more recommendations 720, one or more directed acquisition actions 680 and/or one or more compound results 682.

Discussion next turns to the processor 606, memory 604 and bus 605 of the material analysis system 602. For example, in one or more embodiments, the material analysis system 602 can comprise the processor 606 (e.g., computer processing unit, microprocessor, classical processor, quantum processor and/or like processor). In one or more embodiments, a component associated with material analysis system 602, as described herein with or without reference to the one or more figures of the one or more embodiments, can comprise one or more computer and/or machine readable, writable and/or executable components and/or instructions that can be executed by processor 606 to provide performance of one or more processes defined by such component and/or instruction. In one or more embodiments, the processor 606 can comprise the acquisition component 610, analysis component 612, metric component 614, evaluation component 616, execution component 618, reward component 620 and/or updating component 622.

In one or more embodiments, the material analysis system 602 can comprise the computer-readable memory 604 that can be operably connected to the processor 606. The memory 604 can store computer-executable instructions that, upon execution by the processor 606, can cause the processor 606 and/or one or more other components of the material analysis system 602 (e.g., acquisition component 610, analysis component 612, metric component 614, evaluation component 616, execution component 618, reward component 620 and/or updating component 622) to perform one or more actions. In one or more embodiments, the memory 604 can store computer-executable components (e.g., acquisition component 610, analysis component 612, metric component 614, evaluation component 616, execution component 618, reward component 620 and/or updating component 622).

The material analysis system 602 and/or a component thereof as described herein, can be communicatively, electrically, operatively, optically and/or otherwise coupled to one another via a bus 605. Bus 605 can comprise one or more of a memory bus, memory controller, peripheral bus, external bus, local bus, quantum bus and/or another type of bus that can employ one or more bus architectures. One or more of these examples of bus 605 can be employed.

In one or more embodiments, the material analysis system 602 can be coupled (e.g., communicatively, electrically, operatively, optically and/or like function) to one or more external systems (e.g., a non-illustrated electrical output production system, one or more output targets and/or an output target controller), sources and/or devices (e.g., classical and/or quantum computing devices, communication devices and/or like devices), such as via a network. In one or more embodiments, one or more of the components of the material analysis system 602 and/or of the non-limiting system 600 can reside in the cloud, and/or can reside locally in a local computing environment (e.g., at a specified location).

In addition to the processor 606 and/or memory 604 described above, the material analysis system 602 can comprise one or more computer and/or machine readable, writable and/or executable components and/or instructions that, when executed by processor 606, can provide performance of one or more operations defined by such component and/or instruction.

Turning now to the additional components of the material analysis system 602 (e.g., acquisition component 610, analysis component 612, metric component 614, evaluation component 616, execution component 618, reward component 620 and/or updating component 622), generally, the material analysis system 602 can perform a set of processes that can be separated into three steps: initial acquisition and analysis, evaluation and recommendation, and rewarding and updating.

Turning first to the initial acquisition and analysis, detail will be provided regarding the acquisition component 610, analysis component 612 and metric component 614.

Turning first to the acquisition component 610, this component can generally acquire data (e.g., input data 660) for a compound 662, the data defining a first mass spectrometry spectrum 665 for the compound 662. The input data 660 can further comprise one or more compound metrics 664 (e.g., physical phase temperature ranges, physical properties, mechanical properties, chemical properties and/or the like, without being limited thereto).

In one or more embodiments, the input data 660 can comprise data defining one or more initial acquisitions 703 that define and/or identify one or more starting compounds 662 (e.g., starting compounds 662 with which the non-limiting system 600 is to begin an MS-based experiment).

In one or more embodiments, the acquisition component 610 can identify one or more compounds (e.g., at least a first compound 662 and a second compound 662) from the input data (e.g., from a spectrum 665). In one or more other embodiments, an initial compound 662 can instead be provided as an input to the non-limiting system 600.

The input data 660 can be provided in any suitable format (e.g., text, code, numerical, data, metadata, without being limited thereto).

In one or more embodiments, the acquisition component 610 can provide the input data 660 as input to the NN 670 and/or the NN 670 can obtain the input data 660 by one or more other suitable means.

The metric component 614 can obtain and provide, as an input to the NN, one or more metrics of interest 668 related to a compound 662 or type of compound to be analyzed by way an MS-based experiment employing RL-based control provided by the one or more embodiments discussed herein. A metric of interest 668 can be a high-level goal to be achieved by the non-limiting system 600. In one or more embodiments, a metric of interest 668 is not so descriptive as to comprise lower-level acquisition metrics and/or operation settings that more particularly would define one or more stages of an MS-based experiment. For example, a metric of interest 668 can comprise a directive to achieve a certain number of identifications, achieve a maximum number of identifications, perform a selected number of acquisition actions, achieve a selected identification accuracy, perform rapid identification within the confines of a desired sample separation period (i.e. LC gradient length), use a particular experiment technique or methodology, use a particular MS device 650, etc., without being limited thereto.

A metric of interest 668 can be provided by a user entity and/or can be obtained from the input data 660 and/or historical data 642 (e.g., historical compound metrics 644, historical spectra 646 and/or historical acquisition actions 648).

Based on the input data 660, on data on which the NN 670 has already been trained, and/or on historical data 642 from the database 640, the analysis component 612 can generate an input dataset. The input dataset can be a basis of information upon which the NN 670 can provide a recommendation (e.g., upon being directed by the evaluation component 616).

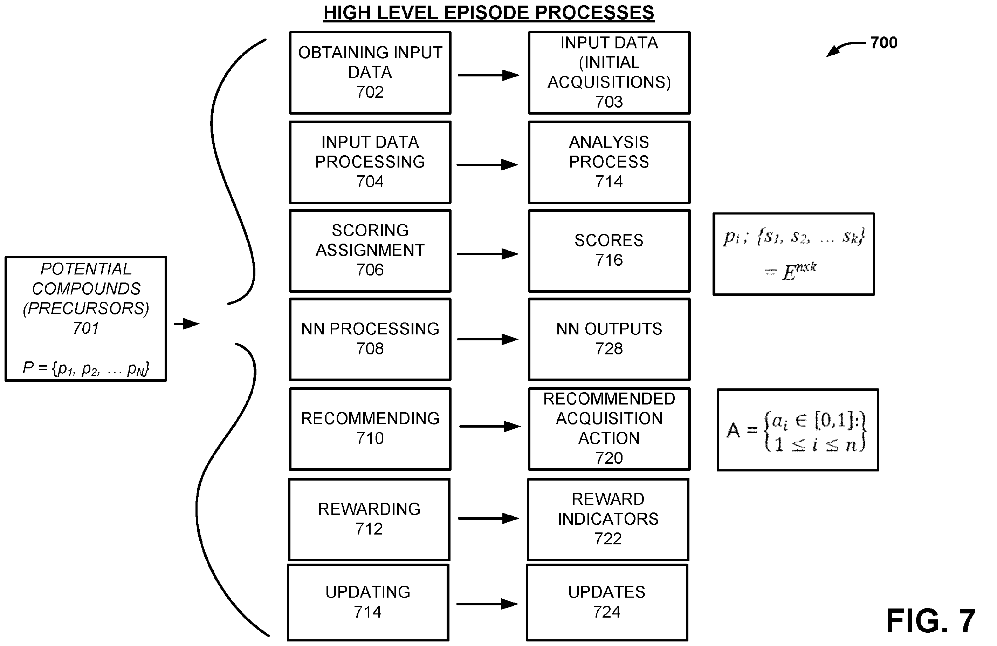

For example, turning to FIG. 7, in addition to still referring to FIG. 6, after the obtaining input data step 702 is completed by the acquisition component 610, input data processing 704 can be performed by the analysis component 712. That is, operation of the analysis process 714 by the analysis component 612 can result in information defining one or more relationships between one or more acquisition metrics 810 (e.g., ion quantity necessary for analysis, analysis temperature, analysis degradation parameters, detection probability, signal intensity, predicted next signal intensity, fit to predicted isotopic cluster, mass accuracy, delta time between estimated current time and expected elution time, and/or whether acquisition was acquire for precursor on las iteration, without being limited thereto) and one or more associated scores 716.

That is, as a result of comparison and/or analysis by the analysis component 612 of the input data 660, historical data 642 and/or data upon which the NN 670 has already been trained, the analysis component 612 can output one or more relationships between different aspects of these data for each potential compound (e.g., precursor $P = \{p_1, p_2, ... p_N\}$) to be analyzed and/or identified by the non-limiting system 600 with regards to an MS-based experiment (e.g., a multi-stage MS-based experiment).

It is noted that the analysis process 714 can be based on, such as performed by the analysis component 612 within the confines of, the requested one or more metrics of interest 668.

As a result of the analysis process 714, a scoring assignment 706 can be performed by the analysis component 612. That is, for one or more acquisition metrics 810, for one or more of the potential compounds (e.g., for each acquisition metric for each potential compound), a score 716 can be assigned. That is, scores 716 can be assigned external to the NN 670.

In one or more embodiments, the acquisition metrics 810 and/or scores 716 can be determined by input from a user entity and/or can be obtained from the input data 660 and/or historical data 642.

In one or more embodiments, the scores 716 can be at least partially generated by another NN, trained to generate scores defining relationships between precursors and acquisition metrics 810. In an example, an additional NN can be employed to predict a next intensity point, given a vector of previous intensities, which intensity can be employed as an input (either as initial input data 660 or as an acquisition metric 810). In an example, an additional NN can be employed to estimate whether there is evidence in input data 660 or data resulting from an acquisition action 680.

In one or more embodiments, a set of scores ($p_i$; $\{s_1, s_2, ... s_k\} = E^{nxk}$) can be numbers between (and including) 0 and 1 (e.g., [0,1]), where n is the number of precursors being considered and k is the number of scores per precursor. The scores could take any values, but most often could be normalized to a range [0, 1], or [-1, 1], or regularized by dividing by an expected or measured mean and dividing by an expected or measured standard deviation. In general, the scores are a description of the environment, in the RL parlance, which in conjunction with the reward feedback loop enable the system to output a recommended action.

For example, referring now in addition to the key 850 for neural network action outputs of FIG. 8, the precursor P1 is entering the mass spectrometer, and has k scores associated with it, including a relatively high intensity, a high next predicted intensity, and a low delta time between the current and predicted elution time, without being limited thereto. The RL system previously determined that it was advantageous to acquire a spectrum for P1, and this is denoted in the last score having value 1. Other precursors in this example have scores that could indicate to the RL system a lower probability of their presence, such that acquiring a spectrum for them is not advantageous, such as lower intensity, worse mass accuracy, and a large delta between the current and predicted elution times.

Based on these inputs the NN outputs an action $a_i$ for each precursor, which in some embodiments could take the value of 0 to indicate no acquisition for a precursor, and 1 to indicate to acquire a spectrum for the precursor. Alternatively the action output could be a continuous variable between 0 and 1, such that 0 indicates no acquisition, while values greater than 0 indicate that a spectrum is to be acquired with a certain amount of accumulation time, for example with an accumulation time of $a_i \times t_{max}$, where $t_{max}$ is a maximum accumulation time. An acquisition acquired with 1.0 could take longer to acquire than an acquisition with 0.1, but could have higher quality (e.g., higher precision).

In one or more embodiments, the one or more associated scores 716 can comprise and/or define one or more probabilities that one or more thresholds corresponding to the one or more acquisition metrics (e.g., acquisition metrics 810) will be satisfied by a mass spectrometry action performed for a compound or type of compound. As used herein, the term "satisfied" can mean reaching or exceeding of a threshold. As used herein, one or more, such as each, acquisition metric 810 can have associated therewith a threshold. For example, referring to FIG. 8, the thresholds can comprise a threshold for fit, signal intensity, mass accuracy, detection probability, delta time, etc.

Referring still to FIG. 8, based on the scores 716 and acquisition metrics 810, a dataset matrix, such as an exemplary scoring matrix 800, can be generated by the analysis component 612. As illustrated at FIG. 8, the matrix 800 comprises a set of acquisition metrics 810 along a vertical side (e.g., rows) and a set of precursors (e.g., potential compounds to acquire/identify) along a horizontal side (e.g., columns).

While illustrated at FIG. 8 as a matrix (or table), the resulting data (e.g., acquisition metrics 810, scores 716, and relationships therebetween) can be stored in any suitable format for use by the NN 670. This resulting data can be stored at the memory 604 and/or at any other suitable location communicatively couplable to the non-limiting system 600.

In one or more embodiments, the input dataset (e.g., dataset matrix) can be pre-generated and the NN 670 can be already trained on the input dataset. That is, the input dataset can comprise one or more acquisition metrics (e.g., acquisition metrics 810) for a compound 662 and/or type of compound, and one or more associated scores 716 that are associated with the one or more acquisition metrics.

In one or more embodiments, a pre-generated or generated input dataset can be updated, e.g., by the analysis component 612, such as by a direction from the metric component 614, to take into account a metric of interest 668. That is, for example, as a result of a metric of interest 668, the acquisition metrics 810 can be expanded, altered, and/or reduced (e.g., the list of acquisition metrics 810 at table 800 can be changed).

In one or more embodiments, the metric component 614 and/or the analysis component 612 can change, modify, update and/or generate the input dataset dimensions based on one or more metrics of interest 668. For example, a user entity submitting a metric of interest 668 may desire to focus on a particular high-level goal defined by the metric of interest. Based on that goal, a score 716 can be updated, or an acquisition metric 810 can be added or deleted, for example, and without being limited thereto. In another example, a precursor can be more or less important based on a metric of interest 668.

Based on the scores 716 (such as based on an aggregation or other calculation employing the scores 716), and guided by the one or more metrics of interest 668, the NN can perform NN processing 708, resulting in one or more NN outputs 728. The NN outputs 728 can be in any suitable format such as can be employed by the material analysis system 602. For example, the one or more NN outputs 728 can define a recommended acquisition action 720 which can itself be defined by and/or comprise one or more operation settings (e.g., # of ions to employ, exposure time, # of fragmentations, etc., without being limited thereto).

Put another way, based on the scores 716 (such as based on an aggregation or other calculation employing the scores 716), guided by the one or more metrics of interest 668, and based on the one or more NN outputs 728, the evaluation component 616 can generate one or more recommendations (e.g., one or more recommended acquisition actions 720). In general, a recommended acquisition action can be and/or comprise a recommendation to perform one or more separations and/or mass spectrometry actions. The one or more separations and/or mass spectrometry actions can directly or indirectly result in an acquisition of a compound.

In one or more embodiments, the NN 670 can be limited to selection from, or directed to preferably select from, an action database 672 of available mass spectrometry actions, which available mass spectrometry actions can be capable of being performed using one or more mass spectrometry devices and/or other separation devices communicatively coupled to the non-limiting system 600.

The action database 672 can be comprised by and/or otherwise communicatively coupled to the material analysis system 602.

As used herein, any one or more of any one or more groups of the historical acquisition actions 648, the recommended acquisition actions 720 and/or the directed acquisition actions 680 can comprise use of a mass spectrometry device or other separation device to directly or indirectly obtain acquisition of the compound. Such an action 648/680/720 can comprise, but is not limited to, any one or more of a fragmentation acquisition, an MSn acquisition, selection of a particular mass spectrometry device, selection of a particular separation device, selection of a device operation setting, selection of a time range for performance of an acquisition, selection of a quantity of ions to employ, selection of an ion application schedule and/or pattern to employ, selection of a two or more acquisitions in parallel, selection of back-to-back acquisitions, and/or selection of a reagent.

For example, in one or more embodiments, the NN 670 can recommend use of a fragmentation-based acquisition.

For another example, in one or more embodiments, the NN 670 can recommend an MS2 or MS3 acquisition.

For further example, turning to FIG. 8, relative to focus on an acquisition metric of predicted next signal intensity, an acquisition action could be recommended for precursor P1, based on the high score, for precursor P2, also based on the high score, or for acquisitions of two targets (e.g., P1 and P2) based on the two higher scores.

In one or more embodiments, an output of the NN 670/evaluation component 616 can be based on historical data 642 defining degradation of a potential precursor or initial compound 662 prior to the acquisition action 680 being performed. As a result, such input can have a bearing on an operation setting of # of ions to accumulate for an acquisition and/or accumulation time, or isolation width, for example.

A recommended acquisition action 720 can be defined by Equation 1.

$$\text{Equation 1: } A = \{a_i \in [0,1]: 1 \leq i \leq n\},$$

$A$ is a set of actions $a_i$ corresponding to each precursor $p_i$, enumerated over the variable $i$ from precursor $i = 1$ to $n$, and where the actions may take on values in the range of 0 to 1, inclusive.

A recommended acquisition action 720 can be and/or can comprise a translation of one or more NN outputs 728, and thus can itself be defined by and/or comprise one or more operation settings (e.g., # of ions to accumulate, accumulation time, # of fragmentations, etc., without being limited thereto).

A recommended acquisition action 720 (and NN output 728) can satisfy one or more acquisition metrics and/or comprise one or more recommended operation setting. In one or more embodiments, specification can be made of a plurality of targets corresponding to the compound 662, anticipated to result from a fragmentation, and/or corresponding to one or more fragmented compounds from a previous spectrum (e.g., spectrum 665), for which data is to be obtained by performance of the recommended acquisition action 720.

A recommended acquisition action 720 can comprise recommendation for a fragmented acquisition that is a mass spectrometry/mass spectrometry (MS2) acquisition for the compound or a mass spectrometry/-mass spectrometry/mass spectrometry (MS3) acquisition for the compound.

The one or more recommended acquisition metrics and/or operation settings can define recommendation of an amount of a resource (e.g., time, number of ions, device type, particular device, etc.) to employ for the recommended acquisition action 720.

In one or more embodiments, the NN 670, and thus the evaluation component 616, can additionally or alternatively output a directive to perform an acquisition action 680.

In one or more embodiments, one or more types of, or specific, acquisition actions 680 can be pre-approved (e.g., by a user entity or via a template being used) to be automatically performed without additional authorization. In one or more embodiments, one or more types of, or specific, acquisition actions 680 can be indicated as requiring authorization for proceeding with execution. In one or more embodiments, all acquisition actions 680 can be pre-approved.

In one or more embodiments, the NN 670, and thus the evaluation component 616, can additionally or alternatively output two or more recommendations, two or more output directives, and/or a combination of two or more recommendations and output directives at least partially in parallel with one another. As such, operation of the non-limiting system 600 can be scalable.

As a result of an output directive, or as a result of an approved recommendation 720, the execution component 618 can direct execution of the mass spectrometry action (e.g., acquisition action 680) at a mass spectrometer (e.g., MS device 650) and can obtain a mass spectrum result (e.g., comprising a compound result 682) therefrom. Approval can be provided, for example, by a user entity using any suitable input and/or input device to the non-limiting system 600.

Additionally, and/or alternatively, based on an execution of an acquisition action 680, the execution component 618 can obtain one or more data, outputs, outcomes, and/or compound results 682 that result from the performance of the acquisition action 680. In one or more embodiments, an output of an acquisition action 680 can comprise generation one or more spectra, which can be obtained by the execution component 618 and analyzed by the execution component 618 and/or analysis component 612.

Referring now to the reward component 620, this component can generally perform a rewarding process 712 that can generate a reward indicator 722 resulting from the recommendation 720 of a mass spectrometry action and/or resulting from a result of an execution of an acquisition action 680. Put another way, the reward component 620 can evaluate the recommended mass spectrometry action 720/executed acquisition action 680 by generating a reward indicator 722. The reward indicator 722 can be based on satisfaction, or non-satisfaction or partial satisfaction, of at least one of the one or more acquisition metrics 810 of the input dataset and/or based on satisfaction, or non-satisfaction or partial satisfaction, one or more of the metrics of interest 668. That is, more generally, the rewarding process 712 can serve to reinforce outputs (e.g., recommended acquisition actions 720 and directed acquisition actions 680) that meet and/or satisfy one or more acquisition metrics 810 and/-metrics of interest 668.

For example, the reward can be an output of a function, e.g., a state score or action, in connection with the NN being and/or providing nonlinear function module. However, in one or more embodiments, an additional NN can be generated, trained and employed to assist the reward component 620 in generating the reward indicators 722.

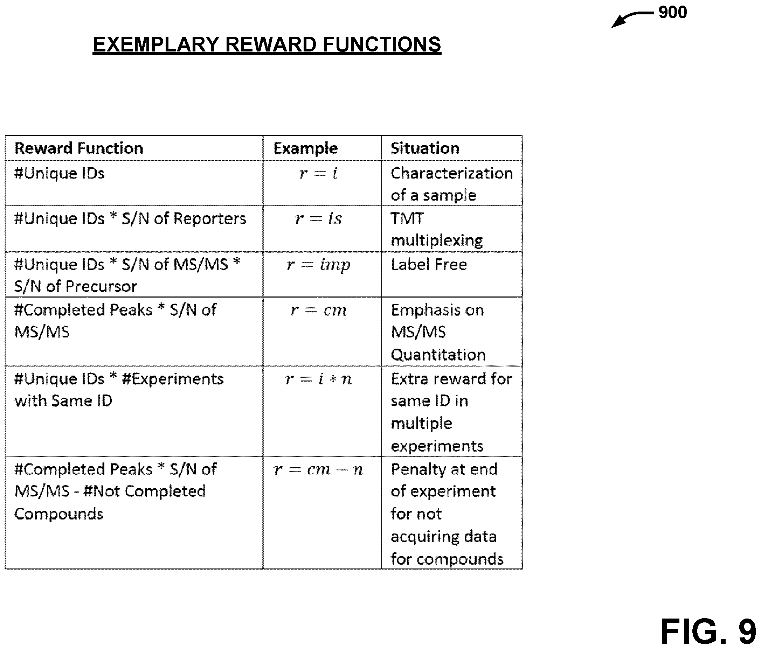

For particular examples, turning briefly to FIG. 9, illustrated is a table 900 of exemplary reward functions. As illustrated, one or more reward indicators r, resulting from one or more reward functions, can be employed relative to any acquisition action and result thereof. As shown, each exemplary reward function has related thereto a situation at which the reward function can be applicable, for ease of comprehension. Exemplary reward functions can include, but are not limited to unique compound identifications, completed peaks, experiments with a same resulting identification and/or any combination thereof.

For example, a simple reward function could comprise the sum of the number of unique peptide detections. As another example, a reward could be dependent on an acquisition metric such as the signal-to-noise-ratio (SNR) of the resultant spectra. In still another example, extra value could be allocated to acquiring multiple spectra that span the elution profile of a compound. This might be achieved through a time-local "peak completion" analysis, or through an even higher-level reward like the precision of measurement over multiple experiments. Value could also be given to detecting the same peptides or other compound type, class and/or group in each iteration of a set of MS-based experiments.

Based on the reward indicator 722, whether positive or negative (e.g., the term "negative" corresponding to a penalty), the updating component 622 can direct self-updating of the NN 670.

In one or more embodiments, this updating process 714 can result in one or more updates 724 comprising updates to weights employed by the NN 670 and/or one or more adjustments to the one or more acquisition metrics 810 based on the particular one or more reward indicators 722 generated.

In one or more embodiments, the updating component 622 can update the NN (e.g., update weights employed by the NN or update relevant acquisition metrics 810) according to a set of reward outputs (e.g., reward indicators 722) amortized over time and obtained based on a plurality of generations (e.g., of recommendations 720) by the evaluation component 616.

Turning now back to FIG. 7, for any MS-based experiment performed by the non-limiting system 600, one or more additional iterations of one or more of the high-level processes 700 (FIG. 7) can be performed. For example, after performance of any recommending process 710, the evaluation component 616 can determine, based on a mass spectrum result (e.g., compound result 682) resulting from a recommendation 720, whether an additional recommendation 720 should be generated to satisfy a metric of interest 668 for the compound. This determination can be made at any suitable frequency depending on the number of parallel recommendations for a same compound or different compounds, or for compounds linked by subsequent fragmentations.

It is noted that the rewarding step 712 and subsequent updating step 714 can be performed at any suitable frequency, such as after a recommending process 710, after determination (e.g., by the execution component 618) of an outcome of an acquisition action 680, etc.

In one or more embodiments, one or more input datasets can be pre-generated and/or one or more NN's 670 can be trained on a compound or a type of compound, such as to provide templates for use by one or more user entities.

For example, envisioned is a general methodology that can be adapted to different types of experiments, labeling technologies, and/or mass or cross section analyzing technologies. Relative to each template, a sample should be understood well enough so that the simulations can match the experimental results such as based on number of ID's, signal-to-noise ration (SNR), measurement precision, etc., without being limited thereto. A template can comprise direction (e.g., as a metric of interest 668) to employ a chromatogram library, gas phase fractionation approach or real actual liquid phase fractionation coupled with traditional DIA or DDA analysis. DDA analysis with narrow isolation windows could be employed, as such could allow for generation of actual MS/MS spectra for all compounds in the sample at all times. Simulation of the LC-MS spectra could in this case entail simply retrieving the appropriate spectra from this chromatogram library. Alternatively, prediction using available software tools could be used to generate MSn spectra and retention time order, and/or experimental information could be used to generate chemical noise and anchor the predicted retention times. In one or more embodiments, a simulation of experiments could utilize data augmentation to prevent overfitting and have the NN 670 experience one or more different states. This data augmentation can take the form of random noise arising from the electrospray or detection process, as well as chromatographic drift and/or autosampler sample delivery errors.

Relative to the general methodology, as mentioned above, a plurality of trained agents (e.g., NN's 670) could be generated and trained covering common use cases such as the parameter space comprising organism, sample type, labelling technology, separation column, separation time, and/or analyzer type. For example, a user entity could specify a compound or compound type of human plasma labeled with TMT 10 plex, using a C18 column, 30-minute gradient, and ion trap analyzer. These specifications could apply as metrics of interest 668 or as requested acquisition metrics 810 and/or operation settings.

In an example of a specific set of samples or compounds that are not fully represented by the plurality of trained agents, setup could comprise a gas-phase fractionation characterization of the sample, followed by training of the agent, which would likely start from the most similar trained agent in an agent library. It could be possible for an agent to be trained in real-time, or to evolve over a series of priming experiments, although in general it may be preferable from a user entity point of view for the same agent to be used for the acquisition of all samples in a set.

Disclosure next turns to a summary of the one or more functions described above as being performed by the non-limiting system 600. That is, consider a LC-MS situation where there is a set of n possible precursors *p* that are known or suspected to be in a sample (e.g., potential precursors *p*).

At a time t, one or more MS or MSn acquisitions can be acquired (e.g., by the acquisition component 610), and the spectra can be used as input to an agent's processing facility (e.g., of an NN 670). For example, an MS1 spectrum or a set of MS/MS spectra can be acquired that span a precursor m/z range.

The system (e.g., analysis component 612) can perform analysis such as cross correlation of these spectra against a stored set of spectra to estimate a reference retention time (e.g., of database 640) and/or perform a search against an experimental or in silico library. Based on the analysis, each precursor can receive a vector of k scores (e.g., by the analysis component 612). The scores can correspond to acquisition metrics 810 such as a detection probability from the database search, a signal intensity, a fit to predicted isotopic cluster, a mass accuracy, a predicted next signal intensity, a delta time between the estimated current time and the expected elution time, and/or whether an acquisition was acquired for the precursor on the last iteration. The scores could include a history of the last m values for each score.

An *n x k* matrix of scores for each precursor (e.g., an exemplary scoring matrix 800) is one way to describe the environment, and data representing such matrix can be input into a neural network 670. An output of the neural network 670 and evaluation component 616 can be a set *A* of actions, one for each of the potential precursors *p*, having a value in [0,1], which values are defined at key 850 for neural network action outputs.

Via the execution component 618, one or more instruments (e.g., MS devices 650) can generate spectra for each of the potential precursors, which can take a certain amount of time. In effect, this changes the state of the environment, as new compounds elute from the column and enter a mass spectrometer being employed (e.g., MS device 650).

Based on one or more outcomes of one or more acquisition actions 680, a reward indicator 722 can be computed (e.g., by the reward component 620) based on a state of the system and on an analysis of the acquired spectra. The reward indicator 722 can be used to update the neural network 670 by the updating component 622.

A next iteration of training and/or use of the material analysis system 602 can then begin by the acquiring (e.g., by the acquisition component 610) of a new batch of exploratory spectra (e.g., DIA spectra). Additionally, or alternatively, analysis of the triggered spectra can be used to update the description of the environment for such next experiment. This can result in one or more changes to an input dataset (e.g., to one or more scores 716, acquisition metrics 810 and/or operation settings).

Relative to the above, it is noted that acquiring exploratory spectra in the form of DIA, even with rather large isolation windows, can have one or more benefits in terms of data completeness. That is, there can be at least some information present for all precursors of interest, even if higher quality acquisitions were never triggered. Alternatively, the exploratory spectra could comprise a set of DIA spectra that change with time, or could comprise narrow isolation targeted MS/MS acquisitions for compounds predicted to elute at a particular time. That is, any kind of acquisition action 680 can trigger another acquisition action 680 and/or another kind of acquisition action 680. Indeed, in one or more embodiments, a choice of exploratory acquisitions can be part of a set of actions available to the system (e.g., to the evaluation component 616).

The triggered spectra could take the form of MS2 spectra or MS3 spectra. For example, the set of actions available to the system (e.g., to the evaluation component 616) could have length 2n, i.e., an option to acquire an MS2 spectrum, and an option to acquire an MS3 spectrum. The system (e.g., evaluation component 616) could determine, based on the available information and the state of the neural network 670, which one or more acquisition actions 680 to recommend, direct and/or to take.

As a result, a benefit of the one or more embodiments described herein can be that the non-limiting system can learn not only simple operations, such as which acquisitions to acquire based on a current environment (e.g., compound environment), but that such decisions could result in a method that takes into account higher level metrics of interest 668, such as acquiring spectra at the top of LC peaks and/or focusing resources on compounds that are likely to be quantifiable in multiple experiments.

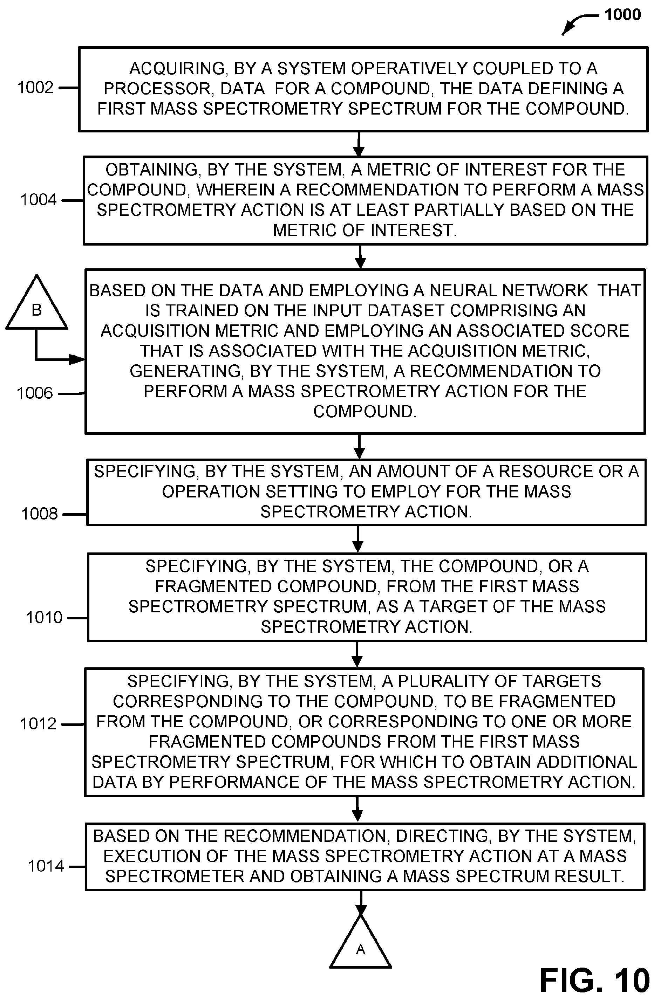

As another summary of the above-described components and functions thereof, referring next to FIGS. 10 and 11, illustrated is a flow diagram of an example, non-limiting method 1000 that can facilitate a process for reinforcement learning-based mass spectrometer control, in accordance with one or more embodiments described herein, such as the non-limiting system 600 of FIG. 6. While the non-limiting method 1000 is described relative to the non-limiting system 600 of FIG. 6, the non-limiting method 1000 can be applicable also to other systems described herein, such as the non-limiting system 500 of FIG. 5. Repetitive description of like elements and/or processes employed in respective embodiments is omitted for sake of brevity.

At 1002, the non-limiting method 1000 can comprise acquiring, by a system operatively coupled to a processor (e.g., acquisition component 610), data (e.g., input data 660) for a compound (e.g., compound 662), the data defining a first mass spectrometry spectrum (e.g., spectrum 665) for the compound.

At 1004, the non-limiting method 1000 can comprise obtaining, by the system (e.g., metric component 614), a metric of interest (e.g., metric of interest 668) for the compound, wherein a recommendation (e.g., recommended acquisition action 720) to perform a mass spectrometry action is at least partially based on the metric of interest.

At 1006, the non-limiting method 1000 can comprise, based on the data and employing a neural network (e.g., NN 670) that is trained on the input dataset comprising an acquisition metric (e.g., compound metric 664, acquisition metric 810 and/or historical compound metric 644) and employing an associated score (e.g., scores 716) that is associated with the acquisition metric, generating, by the system (e.g., evaluation component 616), a recommendation (e.g., recommended acquisition action 720) to perform a mass spectrometry action for the compound.

At 1008, the non-limiting method 1000 can comprise specifying, by the system (e.g., evaluation component 616), an amount of a resource or an operation setting (e.g., time, energy, isolation width) to employ for the mass spectrometry action.

At 1010, the non-limiting method 1000 can comprise specifying, by the system (e.g., evaluation component 616), the compound, or a fragmented compound, from the first mass spectrometry spectrum, as a target of the mass spectrometry action.

1012, the non-limiting method 1000 can comprise specifying, by the system (e.g., evaluation component 616), a plurality of targets corresponding to the compound, to be fragmented from the compound, or corresponding to one or more fragmented compounds from the first mass spectrometry spectrum, for which to obtain additional data by performance of the mass spectrometry action.

At 1014, the non-limiting method 1000 can comprise, based on the recommendation, directing, by the system (e.g., execution component 618), execution of the mass spectrometry action at a mass spectrometer (e.g., MS device 650) and obtaining a mass spectrum result (e.g., compound result 682).

At 1016, the non-limiting method 1000 can comprise generating, by the system (e.g., reward component 620), a reward indicator (e.g., reward indicator 722) resulting from the recommendation of the mass spectrometry action, or from an execution of the mass spectrometry action recommended.

1018, the non-limiting method 1000 can comprise updating, by the system (e.g., updating component 622), one or more weights employed by the NN or making an adjustment to the acquisition metric (e.g., acquisition metrics 810), based on the reward indicator.

At 1020, the non-limiting method 1000 can comprise updating, by the system (e.g., updating component 622), the neural network according to a set of reward indicators amortized over time and obtained based on a plurality of generations of a plurality of recommendations, including the recommendation to perform the mass spectrometry action.

At 1022, the non-limiting method 1000 can comprise determining, by the system (e.g., evaluation component 616), based on the mass spectrum result, whether an additional recommendation should be generated to satisfy the metric of interest for the compound. If yes, the non-limiting method 1000 can proceed back to step 1006. If no, the non-limiting method 1000 can proceed to end.

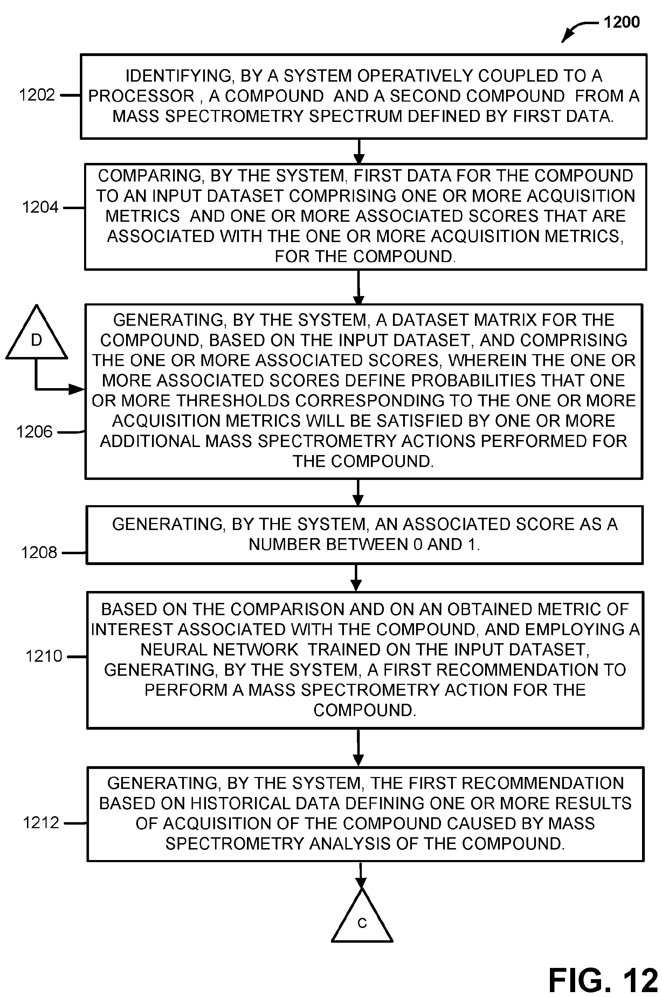

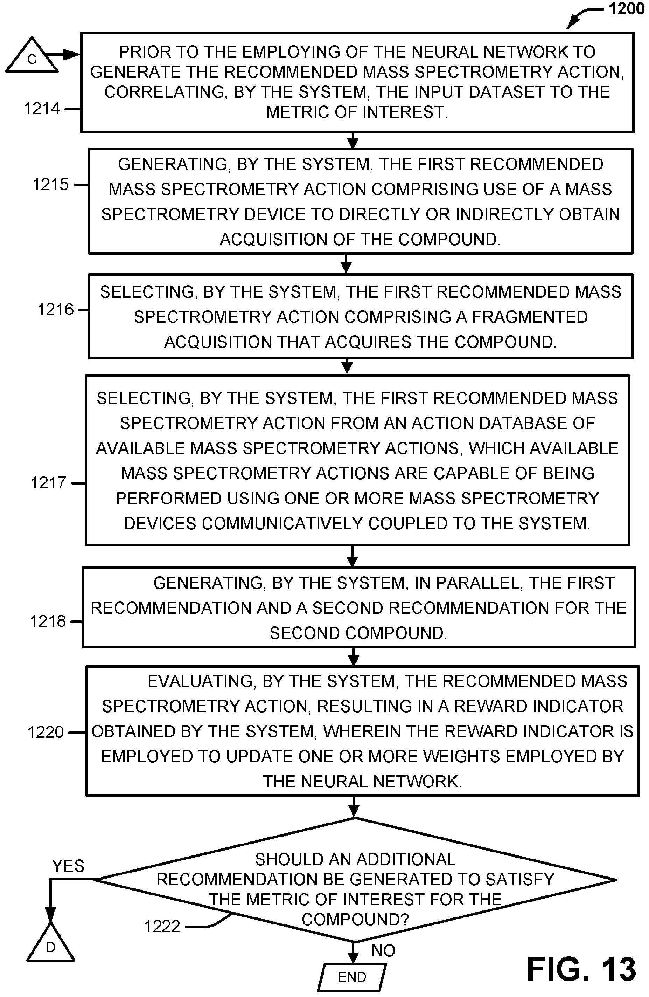

As another summary of the above-described components and functions thereof, referring next to FIGS. 12 and 13, illustrated is a flow diagram of an example, non-limiting method 1200 that can facilitate a process for reinforcement learning-based mass spectrometer control, in accordance with one or more embodiments described herein, such as the non-limiting system 600 of FIG. 6. While the non-limiting method 1200 is described relative to the non-limiting system 600 of FIG. 6, the non-limiting method 1200 can be applicable also to other systems described herein, such as the non-limiting system 500 of FIG. 5. Repetitive description of like elements and/or processes employed in respective embodiments is omitted for sake of brevity.

At 1202, the non-limiting method 1200 can comprise identifying, by a system operatively coupled to a processor (e.g., acquisition component 610), a compound (e.g., compound 662) and a second compound (e.g., another compound 662) from a mass spectrometry spectrum (e.g., spectrum 665) defined by first data (e.g., input data 660).

At 1204, the non-limiting method 1200 can comprise comparing, by the system (e.g., analysis component 612), first data (e.g., input data 660) for the compound to an input data set comprising one or more acquisition metrics (e.g., acquisition metrics 810 and/or historical data 642) and one or more associated scores (e.g., scores 716) that are associated with the one or more acquisition metrics, for the compound.

At 1206, the non-limiting method 1200 can comprise generating, by the system (e.g., analysis component 612), a dataset matrix for the compound (e.g., exemplary scoring matrix 800), based on the input dataset, and comprising the one or more associated scores. In one or more embodiments, the one or more associated scores can define probabilities that one or more thresholds corresponding to the one or more acquisition metrics will be satisfied by one or more additional mass spectrometry actions performed for the compound.

At 1208, the non-limiting method 1200 can comprise generating, by the system (e.g., evaluation component 616), an associated score as a number between 0 and 1.

At 1210, the non-limiting method 1200 can comprise, based on the comparison and on an obtained metric of interest (e.g., metric of interest 668) associated with the compound, and employing a neural network (e.g., NN 670) trained on the input dataset, generating, by the system (e.g., evaluation component 616), a first recommendation (e.g., recommended acquisition action 720) to perform a mass spectrometry action (e.g., acquisition action 680) for the compound.

At 1212, the non-limiting method 1200 can comprise generating, by the system (e.g., evaluation component 616), the first recommendation based on historical data (e.g., historical data 642) defining one or more results of acquisition of the compound caused by mass spectrometry analysis of the compound.

1214, the non-limiting method 1200 can comprise, prior to the employing of the neural network to generate the recommended mass spectrometry action, correlating, by the system (e.g., updating component 622), the input dataset to the metric of interest.

At 1215, the non-limiting method 1200 can comprise generating, by the system (e.g., evaluation component 616 and/or neural network 670), the first recommended mass spectrometry action comprising use of a mass spectrometry device to directly or indirectly obtain acquisition of the compound.

At 1216, the non-limiting method 1200 can comprise selecting, by the system (e.g., evaluation component 616 and/or neural network 670), the first recommended mass spectrometry action comprising a fragmented acquisition that acquires the compound.

At 1217, the non-limiting method 1200 can comprise selecting, by the system (e.g., evaluation component 616 and/or neural network 670), the first recommended mass spectrometry action from an action database (e.g., action database 1272) of available mass spectrometry actions, which available mass spectrometry actions are capable of being performed using one or more mass spectrometry devices communicatively coupled to the system.

At 1218, the non-limiting method 1200 can comprise generating, by the system (e.g., evaluation component 616 and/or neural network 670), in parallel, the first recommendation and a second recommendation (e.g., second recommendation action 720) for the second compound.

1220, the non-limiting method 1200 can comprise evaluating, by the system (e.g., reward component 620), the recommended mass spectrometry action, resulting in a reward indicator (e.g., reward indicator 722) obtained by the system, wherein the reward indicator is employed to update one or more weights employed by the neural network.

At 1222, the non-limiting method 1200 can comprise determining, by the system (e.g., evaluation component 616), based on the mass spectrum result, whether an additional recommendation should be generated to satisfy the metric of interest for the compound. If yes, the non-limiting method 1200 can proceed back to step 1206. If no, the non-limiting method 1200 can proceed to end.

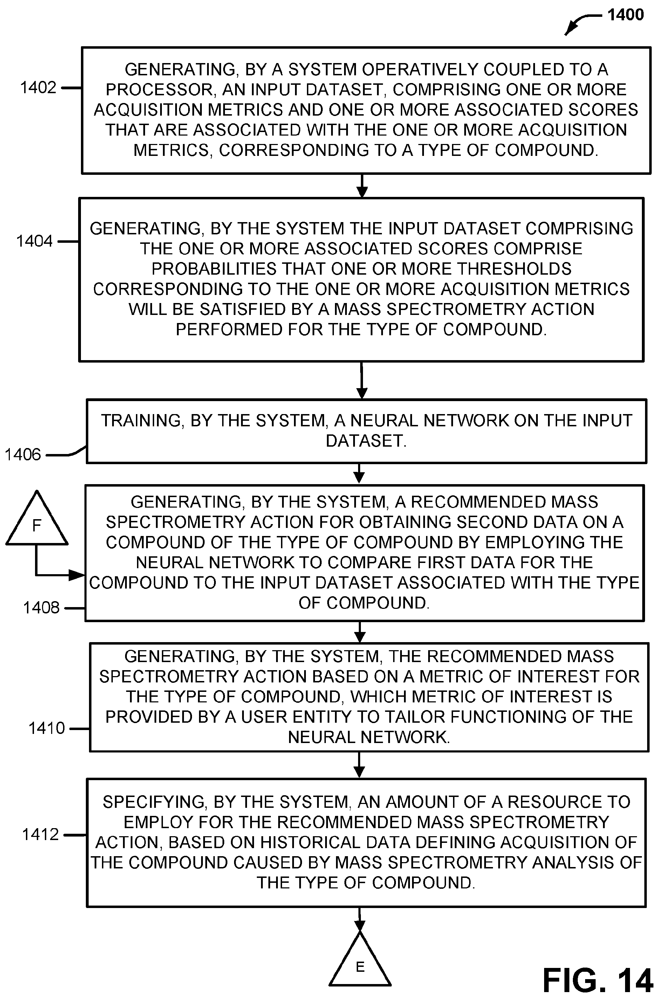

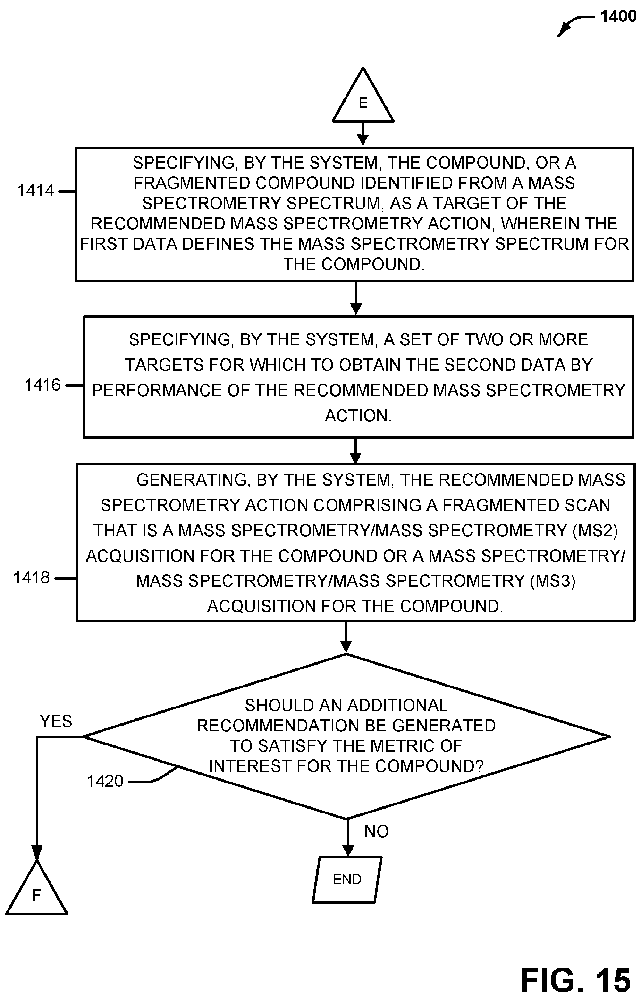

As another summary of the above-described components and functions thereof, referring next to FIGS. 14 and 15, illustrated is a flow diagram of an example, non-limiting method 1400 that can facilitate a process for reinforcement learning-based mass spectrometer control, in accordance with one or more embodiments described herein, such as the non-limiting system 600 of FIG. 6. While the non-limiting method 1400 is described relative to the non-limiting system 600 of FIG. 6, the non-limiting method 1400 can be applicable also to other systems described herein, such as the non-limiting system 500 of FIG. 5. Repetitive description of like elements and/or processes employed in respective embodiments is omitted for sake of brevity.

At 1402, the non-limiting method 1400 can comprise generating, by a system operatively coupled to a processor (e.g., analysis component 612), an input dataset (e.g., exemplary scoring matrix 800), comprising one or more acquisition metrics (e.g., acquisition metrics 810 and/or operation settings) and one or more associated scores (e.g., scores 716) that are associated with the one or more acquisition metrics, corresponding to a type of compound.

At 1404, the non-limiting method 1400 can generating, by the system (e.g., analysis component 612) the input dataset comprising the one or more associated scores comprising probabilities that one or more thresholds corresponding to the one or more acquisition metrics will be satisfied by a mass spectrometry action performed for the type of compound.

At 1406, the non-limiting method 1400 can comprise training, by the system (e.g., updating component 622), a neural network (e.g., neural network 670) on the input dataset.

At 1408, the non-limiting method 1400 can comprise generating, by the system (e.g., evaluation component 616), a recommended mass spectrometry action (e.g., recommended acquisition action 720) for obtaining second data (e.g., compound results 682) on a compound (e.g., a compound 662) of the type of compound by employing the neural network to compare first data (e.g., an input data 660) for the compound to the input dataset (e.g., exemplary scoring matrix 800) associated with the type of compound.

At 1410, the non-limiting method 1400 can comprise generating, by the system (e.g., evaluation component 616), the recommended mass spectrometry action based on a metric of interest (e.g., metric of interest 668) for the type of compound, which metric of interest is provided by a user entity to tailor functioning of the neural network.

1412, the non-limiting method 1400 can comprise specifying, by the system (e.g., evaluation component 616), an amount of a resource (e.g., time, energy, isolation width) to employ for the recommended mass spectrometry action, based on historical data (e.g., historical data 642) defining acquisition of the compound caused by mass spectrometry analysis of the type of compound.

At 1414, the non-limiting method 1400 can comprise specifying, by the system (e.g., evaluation component 616), the compound, or a fragmented compound identified from a mass spectrometry spectrum (e.g., spectrum 665), as a target of the recommended mass spectrometry action, wherein the first data defines the mass spectrometry spectrum for the compound.

At 1416, the non-limiting method 1400 can comprise specifying, by the system (e.g., evaluation component 616), a set of two or more targets for which to obtain the second data by performance of the recommended mass spectrometry action.

At 1418, the non-limiting method 1400 can comprise generating, by the system (e.g., evaluation component 616), the recommended mass spectrometry action comprising a fragmented acquisition that is a mass spectrometry/mass spectrometry (MS2) acquisition for the compound or a mass spectrometry/mass spectrometry/mass spectrometry (MS3) acquisition for the compound.

At 1420, the non-limiting method 1400 can comprise determining, by the system (e.g., evaluation component 616), based on the second data, whether an additional recommended mass spectrometry action should be generated to satisfy the metric of interest for the compound. If yes, the non-limiting method 1400 can proceed back to step 1408. If no, the non-limiting method 1400 can proceed to end.

Additional Summary

For simplicity of explanation, the computer-implemented and non-computer-implemented methodologies provided herein are depicted and/or described as a series of acts. It is to be understood that the subject innovation is not limited by the acts illustrated and/or by the order of acts, for example acts can occur in one or more orders and/or concurrently, and with other acts not presented and described herein. Furthermore, not all illustrated acts can be utilized to implement the computer-implemented and non-computer-implemented methodologies in accordance with the described subject matter. In addition, the computer-implemented and non-computer-implemented methodologies could alternatively be represented as a series of interrelated states via a state diagram or events. Additionally, the computer-implemented methodologies described hereinafter and throughout this specification are capable of being stored on an article of manufacture for transporting and transferring the computer-implemented methodologies to computers. The term article of manufacture, as used herein, is intended to encompass a computer program accessible from any computer-readable device or storage media.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

In summary, one or more systems, computer program products and/or computer-implemented methods provided herein relate to neural network control of mass spectrometry processes. A system can comprise a memory that stores, and a processor that executes, computer executable components. The computer executable components can comprise an acquisition component that acquires data for a compound, the data defining a first mass spectrometry spectrum for the compound, an evaluation component that, based on the data, and employing a neural network that is trained on an input dataset comprising an acquisition metric, and employing an associated score that is associated with the acquisition metric, generates a recommendation to perform a mass spectrometry action for the compound, and an execution component that, based on the recommendation, directs execution of the mass spectrometry action at a mass spectrometer and obtaining a mass spectrum result.

The one or more embodiments disclosed herein can achieve improved performance relative to existing approaches. For example, based on RL training and on historical data on which a NN can be RL trained, a system employing an RL-trained NN, as described herein, can employ better compound-specific acquisition actions than can be merely pre-programmed by an administrative entity. That is, such system can learn to recognize when resources should be spent during current acquisitions and during future recommended or future directed acquisition actions. As noted above, there can be precursor environments where expanded acquisition time can elute a greater number of compounds, or there can be precursor environments where rapid acquisition time can be necessary in view of the large number of compounds entering the mass spectrometer.

Moreover, an embodiment described herein can beneficially provide focus/direction for plural compounds at once, even based on different recommended acquisition actions. That is, an embodiment described herein can employ mixed-optimization techniques to achieve the best results based on the selected high-level goals, such as specificity of analysis, increased precision of compound abundance estimations and/or increased compound detection numbers.

Further, the embodiments described herein can be adapted to different types of experiments, labeling technologies and/or mass or cross section analyzing technologies. That is, depending on a level of sample description or applicable high-level experiment goals, a framework described herein can be adapted to fit the sample. For example, rewards, high level goals, available acquisition actions, etc. can be inputted into the system and into the NN, with the NN being updated to operate within confines of the inputs.

Indeed, in view of the one or more embodiments described herein, a practical application of the one or more systems, computer-implemented methods and/or computer program products described herein can be ability to efficiently maximize outputs of an MS-based experiment, whether the output goals correspond to one or more of specificity of analysis, increased probability of accuracy and/or increased compound detection numbers, among other. This application can be achieved absent specifically setting all acquisition metrics and/or operation settings for the MS-based experiment. Rather, these acquisition metrics and/or operation settings, in addition to one or more acquisition recommendations, can be generated by an RL-trained NN and corresponding material analysis system, as described herein. In these ways, the one or more embodiments described herein can improve upon existing approaches to MS-based experiment control.

These are useful and practical applications of computers, thus providing enhanced (e.g., improved and/or optimized) material analysis and compound data output (e.g., output relating to one or more compounds identified). Overall, such computerized tools can constitute a concrete and tangible technical improvement in the fields of material analysis, and more particularly in material analysis using separation and/or MS techniques.

Furthermore, one or more embodiments described herein can be employed in a real-world system based on the disclosed teachings. For example, as noted above, even in cases where acquisition metrics and/or instrument operation settings are not explicitly input into a system as described herein, the one or more embodiments described herein can perform an MS-based experiment based on an RL-based approach for control of the MS-based experiment. The embodiments disclosed herein thus provide improvements to scientific instrument technology (e.g., improvements in the computer technology supporting such scientific instruments, among other improvements).

Moreover, a device and/or method described herein can be implemented in one or more domains to enable scaled synthesized spectrum output. Indeed, use of a system as described herein can be scalable, such as where plural high-level goals (e.g., metrics of interest) can be facilitated and/or where plural compounds can be focus of current acquisition, at least at a same time as one another.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

One or more embodiments described herein can be, in one or more embodiments, inherently and/or inextricably tied to computer technology and cannot be implemented outside of a computing environment. For example, one or more processes performed by one or more embodiments described herein can more efficiently, and even more feasibly, provide program and/or program instruction execution, such as relative to material analysis based on RL-based control, as compared to existing systems and/or techniques. Systems, computer-implemented methods and/or computer program products providing performance of these processes are of great utility in the fields of material analysis, such as comprising separation and/or MS instruments and cannot be equally practicably implemented in a sensible way outside of a computing environment.

One or more embodiments described herein can employ hardware and/or software to solve problems that are highly technical, that are not abstract, and that cannot be performed as a set of mental acts by a human. For example, a human, or even thousands of humans, cannot efficiently, accurately and/or effectively automatically generate a recommended acquisition action based on an RL-trained approach as the one or more embodiments described herein can provide this process. Moreover, neither can the human mind nor a human with pen and paper conduct one or more of these processes, as conducted by one or more embodiments described herein.

In one or more embodiments, one or more of the processes described herein can be performed by one or more specialized computers (e.g., a specialized processing unit, a specialized classical computer, a specialized quantum computer, a specialized hybrid classical/quantum system and/or another type of specialized computer) to execute defined tasks related to the one or more technologies describe above. One or more embodiments described herein and/or components thereof can be employed to solve new problems that arise through advancements in technologies mentioned above, employment of quantum computing systems, cloud computing systems, computer architecture and/or another technology.

One or more embodiments described herein can be fully operational towards performing one or more other functions (e.g., fully powered on, fully executed and/or another function) while also performing one or more of the one or more operations described herein.

To provide additional summary, a listing of embodiments and features thereof is next provided.

A system, comprising: a memory that stores computer executable components; and a processor that executes the computer executable components stored in the memory, wherein the computer executable components comprise: an acquisition component that acquires data for a compound, the data defining a first mass spectrometry spectrum for the compound; an evaluation component that, based on the data, employing a neural network that is trained on an input dataset comprising one or more acquisition metrics, and employing one or more associated scores that are associated with the one or more acquisition metrics, generates a recommendation to perform a mass spectrometry action for the compound; and an execution component that, based on the recommendation, directs execution of the mass spectrometry action at a mass spectrometer and obtains a mass spectrum result.

The system of the preceding paragraph, wherein the evaluation component further specifies an amount of a resource to employ for the mass spectrometry action.

The system of any preceding paragraph, wherein the evaluation component further specifies the compound, or a fragmented compound, from the first mass spectrometry spectrum, as a target of the mass spectrometry action.

The system of any preceding paragraph, wherein the evaluation component further specifies a plurality of targets corresponding to the compound, to be fragmented from the compound, or corresponding to one or more fragmented compounds from the first mass spectrometry spectrum, for which to obtain additional data by performance of the mass spectrometry action.

The system of any preceding paragraph, further comprising: a metric component that obtains a metric of interest for the compound, wherein the recommendation to perform the mass spectrometry action is at least partially based on the metric of interest.

The system of any preceding paragraph, further comprising: a reward component that generates a reward indicator resulting from the recommendation of the mass spectrometry action, or from an execution of the mass spectrometry action recommended; and an updating component that updates one or more weights employed by the NN or performs an adjustment to the acquisition metric, based on the reward indicator.

The system of any preceding paragraph, further comprising: an updating component that updates the neural network according to a set of reward indicators amortized over time and obtained based on a plurality of recommendations of generations by the evaluation component, including the recommendation to perform the mass spectrometry action.

A computer-implemented method, comprising: comparing, by a system operatively coupled to a processor, first data for a compound to an input data set comprising one or more acquisition metrics and one or more associated scores that are associated with the one or more acquisition metrics, for the compound; and based on the comparison, and on an obtained metric of interest associated with the compound, and employing a neural network trained on the input dataset, generating, by the system, a first recommendation to perform a recommended mass spectrometry action for the compound, wherein the recommended mass spectrometry action comprises use of a mass spectrometry device to obtain acquisition of the compound.

The computer-implemented method of the preceding paragraph, further comprising: identifying, by the system, the compound and a second compound from a mass spectrometry spectrum defined by the first data; and generating in parallel, by the system, the fist recommendation and a second recommendation for the second compound.

The computer-implemented method of any preceding paragraph, wherein the first recommendation is based on historical data defining one or more results of acquisition of the compound caused by mass spectrometry analysis or other separation analysis of the compound.

The computer-implemented method of any preceding paragraph, further comprising: generating, by the system, a dataset matrix for the compound, based on the input dataset, and comprising the one or more associated scores, wherein the one or more associated scores define probabilities that one or more thresholds corresponding to the one or more acquisition metrics will be satisfied by one or more additional mass spectrometry actions performed for the compound.

The computer-implemented method of any preceding paragraph, further comprising: evaluating, by the system, the recommended mass spectrometry action, resulting in a reward indicator obtained by the system, wherein the reward indicator is employed to update one or more weights employed by the neural network.

The computer-implemented method of any preceding paragraph, further comprising: generating, by the system, an associated score as a number between 0 and 1.

The computer-implemented method of any preceding paragraph, further comprising: prior to the employing of the neural network to generate the recommended mass spectrometry action, correlating, by the system, the input dataset to the metric of interest.

The computer-implemented method of any preceding paragraph, further comprising: selecting, by the system, a recommended mass spectrometry action comprising a fragmented acquisition that acquires the compound.

The computer-implemented method of any preceding paragraph, further comprising: selecting, by the system, the recommended mass spectrometry action by the neural network from an action database of available mass spectrometry actions, available mass spectrometry actions are capable of being performed using one or more mass spectrometry devices communicatively coupled to the system.

A computer program product facilitating a process for reinforcement learning-based mass spectrometry control, the computer program product comprising a computer readable storage medium having program instructions embodied therewith, and the program instructions executable by a processor to cause the processor to: generate, by the processor, an input dataset, comprising one or more acquisition metrics and one or more associated scores that are associated with the one or more acquisition metrics, corresponding to a type of compound, wherein the one or more associated scores define probabilities that one or more thresholds corresponding to the one or more acquisition metrics will be satisfied by a mass spectrometry action performed for the type of compound; train, by the processor, a neural network on the input dataset; and generate, by the processor, a recommended mass spectrometry action for obtaining second data on a compound, of the type of compound, by employing the neural network to compare first data for the compound to the input dataset associated with the type of compound.

The computer program product of the preceding paragraph, wherein the generating of the recommended mass spectrometry action is further based on a metric of interest for the type of compound, which metric of interest is provided by a user entity to tailor functioning of the neural network.

The computer program product of any preceding paragraph, wherein the generating of the recommended mass spectrometry action further comprises specifying, by the processor, an amount of a resource to employ for the recommended mass spectrometry action, based on historical data defining acquisition of the compound caused by mass spectrometry analysis of the type of compound.

The computer program product of any preceding paragraph, wherein the first data defines a mass spectrometry spectrum for the compound, and wherein the generating of the recommended mass spectrometry action further comprises specifying, by the processor, the compound, or a fragmented compound identified from the mass spectrometry spectrum, as a target of the recommended mass spectrometry action.

The computer program product of any preceding paragraph, wherein the generating of the recommended mass spectrometry action further comprises specifying, by the system, a set of two or more targets for which to obtain the second data by performance of the recommended mass spectrometry action.

The computer program product of any preceding paragraph, wherein the recommended mass spectrometry action comprises a fragmented acquisition that is a mass spectrometry/mass spectrometry (MS2) acquisition for the compound or a mass spectrometry/mass spectrometry/mass spectrometry (MS3) acquisition for the compound.

Scientific Instrument System Description

Turning next to FIG. 16, a detailed description is provided of additional context for the one or more embodiments described herein at FIGS. 1-15. One or more computing devices implementing any of the scientific instrument modules or methods disclosed herein can be part of a scientific instrument system. FIG. 16 illustrates a block diagram of an example scientific instrument system 1600 in which one or more of the scientific instrument methods or other methods disclosed herein can be performed, in accordance with various embodiments described herein. The scientific instrument modules and methods disclosed herein (e.g., the scientific instrument module 100 of FIG. 1 and the method 200 of FIG. 2) can be implemented by one or more of the scientific instrument 1610, the user local computing device 1620, the service local computing device 1630, and/or the remote computing device 1640 of the scientific instrument system 1600.

Any of the scientific instrument 1610, the user local computing device 1620, the service local computing device 1630, and/or the remote computing device 1640 can include any of the embodiments of the computing device 400 discussed herein with reference to FIG. 4, and any of the scientific instrument 1610, the user local computing device 1620, the service local computing device 1630, and/or the remote computing device 1640 can take the form of any appropriate one or more of the embodiments of the computing device 400 discussed herein with reference to FIG. 4.

One or more of the scientific instrument 1610, the user local computing device 1620, the service local computing device 1630, and/or the remote computing device 1640 can include a processing device 1602, a storage device 1604, and/or an interface device 1606. The processing device 1602 can take any suitable form, including the form of any of the processors 402 discussed herein with reference to FIG. 4. The processing devices 1602 included in different ones of the scientific instrument 1610, the user local computing device 1620, the service local computing device 1630, and/or the remote computing device 1640 can take the same form or different forms. The storage device 1604 can take any suitable form, including the form of any of the storage devices 404 discussed herein with reference to FIG. 4. The storage devices 1604 included in different ones of the scientific instrument 1610, the user local computing device 1620, the service local computing device 1630, and/or the remote computing device 1640 can take the same form or different forms. The interface device 1606 can take any suitable form, including the form of any of the interface devices 406 discussed herein with reference to FIG. 4. The interface devices 1606 included in different ones of the scientific instrument 1610, the user local computing device 1620, the service local computing device 1630, and/or the remote computing device 1640 can take the same form or different forms.

The scientific instrument 1610, the user local computing device 1620, the service local computing device 1630, and/or the remote computing device 1640 can be in communication with other elements of the scientific instrument system 1600 via communication pathways 1608. The communication pathways 1608 can communicatively couple the interface devices 1606 of different ones of the elements of the scientific instrument system 1600, as shown, and can be wired or wireless communication pathways (e.g., in accordance with any of the communication techniques discussed herein with reference to the interface devices 406 of the computing device 400 of FIG. 4). The particular scientific instrument system 1600 depicted in FIG. 16 includes communication pathways between each pair of the scientific instrument 1610, the user local computing device 1620, the service local computing device 1630, and the remote computing device 1640, but this "fully connected" implementation is simply illustrative, and in various embodiments, various ones of the communication pathways 1608 can be omitted. For example, in one or more embodiments, a service local computing device 1630 can omit a direct communication pathway 1608 between its interface device 1606 and the interface device 1606 of the scientific instrument 1610, but can instead communicate with the scientific instrument 1610 via the communication pathway 1608 between the service local computing device 1630 and the user local computing device 1620 and/or the communication pathway 1608 between the user local computing device 1620 and the scientific instrument 1610.

The scientific instrument 1610 can include any appropriate scientific instrument, such as a separation or MS instrument, or other instrument facilitating material analysis.

The user local computing device 1620 can be a computing device (e.g., in accordance with any of the embodiments of the computing device 400 discussed herein) that is local to a user of the scientific instrument 1610. In one or more embodiments, the user local computing device 1620 can also be local to the scientific instrument 1610, but this need not be the case; for example, a user local computing device 1620 that is associated with a home, office or other building associated with a user entity can be remote from, but in communication with, the scientific instrument 1610 so that the user entity can use the user local computing device 1620 to control and/or access data from the scientific instrument 1610. In one or more embodiments, the user local computing device 1620 can be a laptop, smartphone, or tablet device. In one or more embodiments the user local computing device 1620 can be a portable computing device. In one or more embodiments, the user local computing device 1620 can deployed in the field.

The service local computing device 1630 can be a computing device (e.g., in accordance with any of the embodiments of the computing device 400 discussed herein) that is local to an entity that services the scientific instrument 1610. For example, the service local computing device 1630 can be local to a manufacturer of the scientific instrument 1610 or to a third-party service company. In one or more embodiments, the service local computing device 1630 can communicate with the scientific instrument 1610, the user local computing device 1620, and/or the remote computing device 1640 (e.g., via a direct communication pathway 1608 or via multiple "indirect" communication pathways 1608, as discussed above) to receive data regarding the operation of the scientific instrument 1610, the user local computing device 1620, and/or the remote computing device 1640 (e.g., the results of self-tests of the scientific instrument 1610, calibration coefficients used by the scientific instrument 1610, the measurements of sensors associated with the scientific instrument 1610, etc.). In one or more embodiments, the service local computing device 1630 can communicate with the scientific instrument 1610, the user local computing device 1620, and/or the remote computing device 1640 (e.g., via a direct communication pathway 1608 or via multiple "indirect" communication pathways 1608, as discussed above) to transmit data to the scientific instrument 1610, the user local computing device 1620, and/or the remote computing device 1640 (e.g., to update programmed instructions, such as firmware, in the scientific instrument 1610, to initiate the performance of test or calibration sequences in the scientific instrument 1610, to update programmed instructions, such as software, in the user local computing device 1620 or the remote computing device 1640, etc.). A user entity of the scientific instrument 1610 can utilize the scientific instrument 1610 or the user local computing device 1620 to communicate with the service local computing device 1630 to report a problem with the scientific instrument 1610 or the user local computing device 1620, to request a visit from a technician to improve the operation of the scientific instrument 1610, to order consumables or replacement parts associated with the scientific instrument 1610, or for other purposes.

The remote computing device 1640 can be a computing device (e.g., in accordance with any of the embodiments of the computing device 400 discussed herein) that is remote from the scientific instrument 1610 and/or from the user local computing device 1620. In one or more embodiments, the remote computing device 1640 can be included in a datacenter or other large-scale server environment. In one or more embodiments, the remote computing device 1640 can include network-attached storage (e.g., as part of the storage device 1604). The remote computing device 1640 can store data generated by the scientific instrument 1610, perform analyses of the data generated by the scientific instrument 1610 (e.g., in accordance with programmed instructions), facilitate communication between the user local computing device 1620 and the scientific instrument 1610, and/or facilitate communication between the service local computing device 1630 and the scientific instrument 1610.

In one or more embodiments, one or more of the elements of the scientific instrument system 1600 illustrated in FIG. 16 can be omitted. Further, in one or more embodiments, multiple ones of various ones of the elements of the scientific instrument system 1600 of FIG. 16 can be present. For example, a scientific instrument system 1600 can include multiple user local computing devices 1620 (e.g., different user local computing devices 1620 associated with different user entities or in different locations). In another example, a scientific instrument system 1600 can include multiple scientific instruments 1610, all in communication with service local computing device 1630 and/or a remote computing device 1640; in such an embodiment, the service local computing device 1630 can monitor these multiple scientific instruments 1610, and the service local computing device 1630 can cause updates or other information can be "broadcast" to multiple scientific instruments 1610 at the same time. Different ones of the scientific instruments 1610 in a scientific instrument system 1600 can be located close to one another (e.g., in the same room) or farther from one another (e.g., on different floors of a building, in different buildings, in different cities, etc.). In one or more embodiments, a scientific instrument 1610 can be connected to an Internet-of-Things (IoT) stack that allows for command and control of the scientific instrument 1610 through a web-based application, a virtual or augmented reality application, a mobile application, and/or a desktop application. Any of these applications can be accessed by a user entity operating the user local computing device 1620 in communication with the scientific instrument 1610 by the intervening remote computing device 1640. In one or more embodiments, a scientific instrument 1610 can be sold by the manufacturer along with one or more associated user local computing devices 1620 as part of a local scientific instrument computing unit 1612.

In one or more embodiments, different ones of the scientific instruments 1610 included in a scientific instrument system 1600 can be different types of scientific instruments 1610; for example, one scientific instrument 1610 can be an EDS device, while another scientific instrument 1610 can be an analysis device that analyzes results of an EDS device. In some such embodiments, the remote computing device 1640 and/or the user local computing device 1620 can combine data from different types of scientific instruments 1610 included in a scientific instrument system 1600.

Example Operating Environment

FIG. 17 is a schematic block diagram of an operating environment 1700 with which the described subject matter can interact. The operating environment 1700 comprises one or more remote component(s) 1710. The remote component(s) 1710 can be hardware and/or software (e.g., threads, processes, computing devices). In one or more embodiments, remote component(s) 1710 can be a distributed computer system, connected to a local automatic scaling component and/or programs that use the resources of a distributed computer system, via communication framework 1740. Communication framework 1740 can comprise wired network devices, wireless network devices, mobile devices, wearable devices, radio access network devices, gateway devices, femtocell devices, servers, etc.

The operating environment 1700 also comprises one or more local component(s) 1720. The local component(s) 1720 can be hardware and/or software (e.g., threads, processes, computing devices). In one or more embodiments, local component(s) 1720 can comprise an automatic scaling component and/or programs that communicate/use the remote resources 1710 and 1720, etc., connected to a remotely located distributed computing system via communication framework 1740.

One possible communication between a remote component(s) 1710 and a local component(s) 1720 can be in the form of a data packet adapted to be transmitted between two or more computer processes. Another possible communication between a remote component(s) 1710 and a local component(s) 1720 can be in the form of circuit-switched data adapted to be transmitted between two or more computer processes in radio time slots. The operating environment 1700 comprises a communication framework 1740 that can be employed to facilitate communications between the remote component(s) 1710 and the local component(s) 1720, and can comprise an air interface, e.g., interface of a UMTS network, via an LTE network, etc. Remote component(s) 1710 can be operably connected to one or more remote data store(s) 1750, such as a hard drive, solid state drive, subscriber identity module (SIM) card, electronic SIM (eSIM), device memory, etc., that can be employed to store information on the remote component(s) 1710 side of communication framework 1740. Similarly, local component(s) 1720 can be operably connected to one or more local data store(s) 1730, that can be employed to store information on the local component(s) 1720 side of communication framework 1740.

Example Computing Environment

In order to provide additional context for various embodiments described herein, FIG. 18 and the following discussion are intended to provide a brief, general description of a suitable computing environment 1800 in which the various embodiments of the embodiment described herein can be implemented. While the embodiments have been described above in the general context of computer-executable instructions that can run on one or more computers, those skilled in the art will recognize that the embodiments can be also implemented in combination with other program modules and/or as a combination of hardware and software.

Generally, program modules include routines, programs, components, data structures, etc., that perform tasks or implement abstract data types. Moreover, the methods can be practiced with other computer system configurations, including single-processor or multiprocessor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can also be practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, and/or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data, or unstructured data.

Computer-readable storage media can include, but are not limited to, random access memory (RAM), read only memory (ROM), electrically erasable programmable read only memory (EEPROM), flash memory or other memory technology, compact disk read only memory (CD ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible and/or non-transitory media which can be used to store desired information. In this regard, the terms "tangible" or "non-transitory" herein as applied to storage, memory, or computer-readable media, exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries, or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

Referring still to FIG. 18, the example computing environment 1800 which can implement one or more embodiments described herein includes a computer 1802, the computer 1802 including a processing unit 1804, a system memory 1806 and a system bus 1808. The system bus 1808 couples system components including, but not limited to, the system memory 1806 to the processing unit 1804. The processing unit 1804 can be any of various commercially available processors. Dual microprocessors and other multi processor architectures can also be employed as the processing unit 1804.

The system bus 1808 can be any of several types of bus structure that can further interconnect to a memory bus (with or without a memory controller), a peripheral bus, and a local bus using any of a variety of commercially available bus architectures. The system memory 1806 includes ROM 1810 and RAM 1812. A basic input/output system (BIOS) can be stored in a non-volatile memory such as ROM, erasable programmable read only memory (EPROM), EEPROM, which BIOS contains the basic routines that help to transfer information between elements within the computer 1802, such as during startup. The RAM 1812 can also include a high-speed RAM such as static RAM for caching data.

The computer 1802 further includes an internal hard disk drive (HDD) 1814 (e.g., EIDE, SATA), and can include one or more external storage devices 1816 (e.g., a magnetic floppy disk drive (FDD) 1816, a memory stick or flash drive reader, a memory card reader, etc.). While the internal HDD 1814 is illustrated as located within the computer 1802, the internal HDD 1814 can also be configured for external use in a suitable chassis (not shown). Additionally, while not shown in computing environment 1800, a solid-state drive (SSD) could be used in addition to, or in place of, an HDD 1814.

Other internal or external storage can include at least one other storage device 1820 with storage media 1822 (e.g., a solid-state storage device, a nonvolatile memory device, and/or an optical disk drive that can read or write from removable media such as a CD-ROM disc, a DVD, a BD, etc.). The external storage 1816 can be facilitated by a network virtual machine. The HDD 1814, external storage device 1816 and storage device (e.g., drive) 1820 can be connected to the system bus 1808 by an HDD interface 1824, an external storage interface 1826 and a drive interface 1828, respectively.

The drives and their associated computer-readable storage media provide nonvolatile storage of data, data structures, computer-executable instructions, and so forth. For the computer 1802, the drives and storage media accommodate the storage of any data in a suitable digital format. Although the description of computer-readable storage media above refers to respective types of storage devices, other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1812, including an operating system 1830, one or more application programs 1832, other program modules 1834 and program data 1836. All or portions of the operating system, applications, modules, and/or data can also be cached in the RAM 1812. The systems and methods described herein can be implemented utilizing various commercially available operating systems or combinations of operating systems.

Computer 1802 can optionally comprise emulation technologies. For example, a hypervisor (not shown) or other intermediary can emulate a hardware environment for operating system 1830, and the emulated hardware can optionally be different from the hardware illustrated in FIG. 18. In such an embodiment, operating system 1830 can comprise one virtual machine (VM) of multiple VMs hosted at computer 1802. Furthermore, operating system 1830 can provide runtime environments, such as the Java runtime environment or the .NET framework, for applications 1832. Runtime environments are consistent execution environments that allow applications 1832 to run on any operating system that includes the runtime environment. Similarly, operating system 1830 can support containers, and applications 1832 can be in the form of containers, which are lightweight, standalone, executable packages of software that include, e.g., code, runtime, system tools, system libraries and settings for an application.

Further, computer 1802 can be enabled with a security module, such as a trusted processing module (TPM). For instance, with a TPM, boot components hash next in time boot components, and wait for a match of results to secured values, before loading a next boot component. This process can take place at any layer in the code execution stack of computer 1802, e.g., applied at the application execution level or at the operating system (OS) kernel level, thereby enabling security at any level of code execution.

A user entity can enter commands and information into the computer 1802 through one or more wired/-wireless input devices, e.g., a keyboard 1838, a touch screen 1840, and a pointing device, such as a mouse 1842. Other input devices (not shown) can include a microphone, an infrared (IR) remote control, a radio frequency (RF) remote control, or other remote control, a joystick, a virtual reality controller and/or virtual reality headset, a game pad, a stylus pen, an image input device, e.g., camera, a gesture sensor input device, a vision movement sensor input device, an emotion or facial detection device, a biometric input device, e.g., fingerprint or iris scanner, or the like. These and other input devices are often connected to the processing unit 1804 through an input device interface 1844 that can be coupled to the system bus 1808, but can be connected by other interfaces, such as a parallel port, an IEEE 1394 serial port, a game port, a USB port, an IR interface, a BLUETOOTH® interface, etc.

A monitor 1846 or other type of display device can also be connected to the system bus 1808 via an interface, such as a video adapter 1848. In addition to the monitor 1846, a computer typically includes other peripheral output devices (not shown), such as speakers, printers, etc.

The computer 1802 can operate in a networked environment using logical connections via wired and/or wireless communications to one or more remote computers, such as a remote computer 1850. The remote computer 1850 can be a workstation, a server computer, a router, a personal computer, portable computer, microprocessor-based entertainment appliance, a peer device or other common network node, and typically includes many or all of the elements described relative to the computer 1802, although, for purposes of brevity, only a memory/storage device 1852 is illustrated. The logical connections depicted include wired/wireless connectivity to a local area network (LAN) 1854 and/or larger networks, e.g., a wide area network (WAN) 1856. Such LAN and WAN networking environments are commonplace in offices and companies, and facilitate enterprise-wide computer networks, such as intranets, all of which can connect to a global communications network, e.g., the Internet.

When used in a LAN networking environment, the computer 1802 can be connected to the local network 1854 through a wired and/or wireless communication network interface or adapter 1858. The adapter 1858 can facilitate wired or wireless communication to the LAN 1854, which can also include a wireless access point (AP) disposed thereon for communicating with the adapter 1858 in a wireless mode.

When used in a WAN networking environment, the computer 1802 can include a modem 1860 or can be connected to a communications server on the WAN 1856 via other means for establishing communications over the WAN 1856, such as by way of the Internet. The modem 1860, which can be internal or external and a wired or wireless device, can be connected to the system bus 1808 via the input device interface 1844. In a networked environment, program modules depicted relative to the computer 1802 or portions thereof, can be stored in the remote memory/storage device 1852. The network connections shown are example and other means of establishing a communications link between the computers can be used.

When used in either a LAN or WAN networking environment, the computer 1802 can access cloud storage systems or other network-based storage systems in addition to, or in place of, external storage devices 1816 as described above. Generally, a connection between the computer 1802 and a cloud storage system can be established over a LAN 1854 or WAN 1856 e.g., by the adapter 1858 or modem 1860, respectively. Upon connecting the computer 1802 to an associated cloud storage system, the external storage interface 1826 can, with the aid of the adapter 1858 and/or modem 1860, manage storage provided by the cloud storage system as it would other types of external storage. For instance, the external storage interface 1826 can be configured to provide access to cloud storage sources as if those sources were physically connected to the computer 1802.

The computer 1802 can be operable to communicate with any wireless devices or entities operatively disposed in wireless communication, e.g., a printer, scanner, desktop and/or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH® wireless technologies. Thus, the communication can be a defined structure as with an existing network or simply an ad hoc communication between at least two devices.

Additional Information

The embodiments described herein can be directed to one or more of a system, a method, an apparatus and/or a computer program product at any possible technical detail level of integration. The computer program product can include a computer readable storage medium (or media) having computer readable program instructions thereon for causing a processor to carry out aspects of the one or more embodiments described herein. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a superconducting storage device and/or any suitable combination of the foregoing. A non-exhaustive list of more specific examples of the computer readable storage medium can also include the following:

a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon and/or any suitable combination of the foregoing. A computer readable storage medium, as used herein, is not to be construed as being transitory signals per se, such as radio waves and/or other freely propagating electromagnetic waves, electromagnetic waves propagating through a waveguide and/or other transmission media (e.g., light pulses passing through a fiber-optic cable), and/or electrical signals transmitted through a wire.

Computer readable program instructions described herein can be downloaded to respective computing/processing devices from a computer readable storage medium and/or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of the one or more embodiments described herein can be assembler instructions, instruction-set-architecture (ISA) instructions, machine instructions, machine dependent instructions, microcode, firmware instructions, state-setting data, configuration data for integrated circuitry, and/or source code and/or object code written in any combination of one or more programming languages, including an object oriented programming language such as Smalltalk, C++ or the like, and/or procedural programming languages, such as the "C" programming language and/or similar programming languages. The computer readable program instructions can execute entirely on a computer, partly on a computer, as a stand-alone software package, partly on a computer and/or partly on a remote computer or entirely on the remote computer and/or server. In the latter scenario, the remote computer can be connected to a computer through any type of network, including a local area network (LAN) and/or a wide area network (WAN), and/or the connection can be made to an external computer (for example, through the Internet using an Internet Service Provider). In one or more embodiments, electronic circuitry including, for example, programmable logic circuitry, field-programmable gate arrays (FPGA) and/or programmable logic arrays (PLA) can execute the computer readable program instructions by utilizing state information of the computer readable program instructions to personalize the electronic circuitry, in order to perform aspects of the one or more embodiments described herein.

Aspects of the one or more embodiments described herein are described with reference to flowchart illustrations and/or block diagrams of methods, apparatus (systems), and computer program products according to one or more embodiments described herein. It will be understood that each block of the flowchart illustrations and/or block diagrams, and combinations of blocks in the flowchart illustrations and/or block diagrams, can be implemented by computer readable program instructions. These computer readable program instructions can be provided to a processor of a general-purpose computer, special purpose computer and/or other programmable data processing apparatus to produce a machine, such that the instructions, which execute via the processor of the computer or other programmable data processing apparatus, can create means for implementing the functions/acts specified in the flowchart and/or block diagram block or blocks. These computer readable program instructions can also be stored in a computer readable storage medium that can direct a computer, a programmable data processing apparatus and/or other devices to function in a particular manner, such that the computer readable storage medium having instructions stored therein can comprise an article of manufacture including instructions which can implement aspects of the function/act specified in the flowchart and/or block diagram block or blocks. The computer readable program instructions can also be loaded onto a computer, other programmable data processing apparatus and/or other device to cause a series of operational acts to be performed on the computer, other programmable apparatus and/or other device to produce a computer implemented process, such that the instructions which execute on the computer, other programmable apparatus and/or other device implement the functions/acts specified in the flowchart and/or block diagram block or blocks.

The flowcharts and block diagrams in the figures illustrate the architecture, functionality and/or operation of possible implementations of systems, computer-implementable methods and/or computer program products according to one or more embodiments described herein. In this regard, each block in the flowchart or block diagrams can represent a module, segment and/or portion of instructions, which comprises one or more executable instructions for implementing the specified logical function. In one or more alternative implementations, the functions noted in the blocks can occur out of the order noted in the Figures. For example, two blocks shown in succession can be executed substantially concurrently, and/or the blocks can sometimes be executed in the reverse order, depending upon the functionality involved. It will also be noted that each block of the block diagrams and/or flowchart illustration, and/or combinations of blocks in the block diagrams and/or flowchart illustration, can be implemented by special purpose hardware-based systems that can perform the specified functions and/or acts and/or carry out one or more combinations of special purpose hardware and/or computer instructions.

While the subject matter has been described above in the general context of computer-executable instructions of a computer program product that runs on a computer and/or computers, those skilled in the art will recognize that the one or more embodiments herein also can be implemented at least partially in parallel with one or more other program modules. Generally, program modules include routines, programs, components and/or data structures that perform particular tasks and/or implement particular abstract data types. Moreover, the aforedescribed computer-implemented methods can be practiced with other computer system configurations, including single-processor and/or multiprocessor computer systems, mini-computing devices, mainframe computers, as well as computers, hand-held computing devices (e.g., PDA, phone), and/or microprocessor-based or programmable consumer and/or industrial electronics. The illustrated aspects can also be practiced in distributed computing environments in which tasks are performed by remote processing devices that are linked through a communications network. However, one or more, if not all aspects of the one or more embodiments described herein can be practiced on stand-alone computers. In a distributed computing environment, program modules can be located in both local and remote memory storage devices.

As used in this application, the terms "component," "system," "platform" and/or "interface" can refer to and/or can include a computer-related entity or an entity related to an operational machine with one or more specific functionalities. The entities described herein can be either hardware, a combination of hardware and software, software, or software in execution. For example, a component can be, but is not limited to being, a process running on a processor, a processor, an object, an executable, a thread of execution, a program and/or a computer. By way of illustration, both an application running on a server and the server can be a component. One or more components can reside within a process and/or thread of execution and a component can be localized on one computer and/or distributed between two or more computers. In another example, respective components can execute from various computer readable media having various data structures stored thereon. The components can communicate via local and/or remote processes such as in accordance with a signal having one or more data packets (e.g., data from one component interacting with another component in a local system, distributed system and/or across a network such as the Internet with other systems via the signal). As another example, a component can be an apparatus with specific functionality provided by mechanical parts operated by electric or electronic circuitry, which is operated by a software and/or firmware application executed by a processor. In such a case, the processor can be internal and/or external to the apparatus and can execute at least a part of the software and/or firmware application. As yet another example, a component can be an apparatus that provides specific functionality through electronic components without mechanical parts, where the electronic components can include a processor and/or other means to execute software and/or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Moreover, articles "a" and "an" as used in the subject specification and annexed drawings should generally be construed to mean "one or more" unless specified otherwise or clear from context to be directed to a singular form. As used herein, the terms "example" and/or "exemplary" are utilized to mean serving as an example, instance, or illustration. For the avoidance of doubt, the subject matter described herein is not limited by such examples. In addition, any aspect or design described herein as an "example" and/or "exemplary" is not necessarily to be construed as preferred or advantageous over other aspects or designs, nor is it meant to preclude equivalent exemplary structures and techniques known to those of ordinary skill in the art.

As it is employed in the subject specification, the term "processor" can refer to substantially any computing processing unit and/or device comprising, but not limited to, single-core processors; single-processors with software multithread execution capability; multi-core processors; multi-core processors with software multithread execution capability; multi-core processors with hardware multithread technology; parallel platforms; and/or parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, and/or any combination thereof designed to perform the functions described herein. Further, processors can exploit nano-scale architectures such as, but not limited to, molecular and quantum-dot based transistors, switches and/or gates, in order to optimize space usage and/or to enhance performance of related equipment. A processor can be implemented as a combination of computing processing units.

Herein, terms such as "store," "storage," "data store," data storage," "database," and substantially any other information storage component relevant to operation and functionality of a component are utilized to refer to "memory components," entities embodied in a "memory," or components comprising a memory. Memory and/or memory components described herein can be either volatile memory or nonvolatile memory or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory and/or nonvolatile random-access memory (RAM) (e.g., ferroelectric RAM (FeRAM). Volatile memory can include RAM, which can act as external cache memory, for example. By way of illustration and not limitation, RAM can be available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM) and/or Rambus dynamic RAM (RDRAM). Additionally, the described memory components of systems and/or computer-implemented methods herein are intended to include, without being limited to including, these and/or any other suitable types of memory.

What has been described above includes mere examples of systems and computer-implemented methods. It is, of course, not possible to describe every conceivable combination of components and/or computer-implemented methods for purposes of describing the one or more embodiments, but one of ordinary skill in the art can recognize that many further combinations and/or permutations of the one or more embodiments are possible. Furthermore, to the extent that the terms "includes," "has," "possesses," and the like are used in the detailed description, claims, appendices and/or drawings such terms are intended to be inclusive in a manner similar to the term "comprising" as "comprising" is interpreted when employed as a transitional word in a claim.

The descriptions of the various embodiments can use the phrases "an embodiment," "various embodiments," "one or more embodiments" and/or "some embodiments," each of which can refer to one or more of the same or different embodiments.

The descriptions of the various embodiments have been presented for purposes of illustration but are not intended to be exhaustive or limited to the embodiments described herein. Many modifications and variations will be apparent to those of ordinary skill in the art without departing from the scope and spirit of the described embodiments. The terminology used herein was chosen to best explain the principles of the embodiments, the practical application and/or technical improvement over technologies found in the marketplace, and/or to enable others of ordinary skill in the art to understand the embodiments described herein.

## Claims

1. A computer-implemented method, comprising:

   comparing, by a system operatively coupled to a processor, first data for a compound to an input data set comprising one or more acquisition metrics and one or more associated scores that are associated with the one or more acquisition metrics, for the compound; and
   based on the comparison, and on an obtained metric of interest associated with the compound, and employing a neural network trained on the input dataset, generating, by the system, a first recommendation to perform a recommended mass spectrometry action for the compound, wherein the recommended mass spectrometry action comprises use of a mass spectrometry device to obtain acquisition of the compound.

2. The computer-implemented method of claim 1, further comprising:

   identifying, by the system, the compound and a second compound from a mass spectrometry spectrum defined by the first data; and
   generating in parallel, by the system, the fist recommendation and a second recommendation for the second compound.

3. The computer-implemented method of claim 1, wherein the first recommendation is based on historical data defining one or more results of acquisition of the compound caused by mass spectrometry analysis or other separation analysis of the compound.

4. The computer-implemented method of claim 1, further comprising:
   generating, by the system, a dataset matrix for the compound, based on the input dataset, and comprising the one or more associated scores, wherein the one or more associated scores define probabilities that one or more thresholds corresponding to the one or more acquisition metrics will be satisfied by one or more additional mass spectrometry actions performed for the compound.

5. The computer-implemented method of claim 1, further comprising:
   evaluating, by the system, the recommended mass spectrometry action, resulting in a reward indicator obtained by the system, wherein the reward indicator is employed to update one or more weights employed by the neural network.

6. The computer-implemented method of claim 1, further comprising:
   generating, by the system, an associated score as a number between 0 and 1.

7. The computer-implemented method of claim 1, further comprising:
   prior to the employing of the neural network to generate the recommended mass spectrometry action, correlating, by the system, the input dataset to the metric of interest.

8. The computer-implemented method of claim 1, further comprising:
   selecting, by the system, a recommended mass spectrometry action comprising a fragmented acquisition that acquires the compound.

9. The computer-implemented method of claim 1, further comprising:
   selecting, by the system, the recommended mass spectrometry action by the neural network from an action database of available mass spectrometry actions, available mass spectrometry actions are capable of being performed using one or more mass spectrometry devices communicatively coupled to the system.

10. A computer program comprising program code which, when executed by a processor, carries out the computer implemented method of any one of the preceding claims.

11. A system comprising a memory in which is stored the computer program of claim 10, and a processor configured to execute the computer program stored upon the memory.

SCIENTIFIC INSTRUMENT MODULE 100

FIRST (ACQUISITION ANALYSIS) LOGIC 102

SECOND (SCORE ASSIGNMENT) LOGIC 104

THIRD (NEURAL NETWORK EVALUATION) LOGIC 106

FOURTH (ACQUISITION ACTION DETERMINATION) LOGIC 108

FIFTH (NEURAL NETWORK PERFORMANCE EVALUATION) LOGIC 110

**FIG. 1**

200

```
┌─────────────────────────────┐
│   PERFORM FIRST OPERATIONS: │
│    IDENTIFYING COMPOUNDS    │
│             202             │
└─────────────────────────────┘
              │
              ▼
┌─────────────────────────────┐
│  PERFORM SECOND OPERATIONS: │
│     ASSIGNING SCORES TO     │
│     PROCESSING METRICS      │
│             204             │
└─────────────────────────────┘
              │
              ▼
┌─────────────────────────────┐
│   PERFORM THIRD OPERATIONS: │
│   PERFORMING NN EVALUATION  │
│             206             │
└─────────────────────────────┘
              │
              ▼
┌─────────────────────────────┐
│  PERFORM FOURTH OPERATIONS: │
│   DETERMINING RECOMMENDED    │
│     ACQUISITION ACTION      │
│             208             │
└─────────────────────────────┘
              │
              ▼
┌─────────────────────────────┐
│   PERFORM FIFTH OPERATIONS: │
│  PERFORMING NN PERFORMANCE  │
│         EVALUATION          │
│             210             │
└─────────────────────────────┘
```

# FIG. 2

FIG. 3

COMPUTING DEVICE 400

PROCESSOR
402

BATTERY/POWER
CIRCUITRY
408

STORAGE DEVICE
404

DISPLAY DEVICE
410

INTERFACE DEVICE
406

OTHER I/O DEVICES
412

EP 4 542 553 A1

FIG. 4

FIG. 5

EP 4 542 553 A1

FIG. 6

## HIGH LEVEL EPISODE PROCESSES

700

POTENTIAL
COMPOUNDS
(PRECURSORS)
701

$P = \{p_1, p_2, \dots p_N\}$

OBTAINING INPUT
DATA
702

→

INPUT DATA
(INITIAL
ACQUISITIONS)
703

INPUT DATA
PROCESSING
704

→

ANALYSIS
PROCESS
714

SCORING
ASSIGNMENT
706

→

SCORES
716

$$p_i ; \{s_1, s_2, \dots s_k\} = E^{n \times k}$$

NN PROCESSING
708

→

NN OUTPUTS
728

RECOMMENDING
710

→

RECOMMENDED
ACQUISITION
ACTION
720

$$A = \left\{ \begin{array}{l} a_i \in [0,1]: \\ 1 \leq i \leq n \end{array} \right\}$$

REWARDING
712

→

REWARD
INDICATORS
722

UPDATING
714

→

UPDATES
724

**FIG. 7**

EP 4 542 553 A1

## EXEMPLARY SCORING MATRIX 800

### PRECURSORS

**ACQUISITION METRICS (810)**

| | P1 | P2 | P3 | ... | PN |
|---|---|---|---|---|---|
| Detection Probability | 0.1 | 0.05 | 0.01 | | 0 |
| Signal Intensity | 10000 | 1000 | 100 | | 10 |
| Predicted Next Signal Intensity | 20000 | 500 | 103 | | 3 |
| Fit to Predicted Isotopic Cluster | 0.95 | 0.1 | 0.06 | | 0.01 |
| Mass Accuracy | 0.99 | 0.045 | 0.1 | | 0.09 |
| Delta Time Between Estimated Current Time and Expected Elution Time | 0.01 | 5.1 | 2.2 | | 8.4 |
| Acquisition was Acquired for Precursor on Last Iteration | 1 | 0 | 0 | | 0 |

### KEY 850 FOR NEURAL NETWORK ACTION OUTPUTS

**Actions [0,1]        Potential Acquisition Action**

| Actions [0,1] | Potential Acquisition Action |
|---|---|
| 0 | Do not acquire spectrum |
| 0<a<1 | Acquire Spectrum with a times the # of ions |
| 1 | Acquire Spectrum |

**FIG. 8**

EP 4 542 553 A1

## EXEMPLARY REWARD FUNCTIONS

| Reward Function | Example | Situation |
|---|---|---|
| #Unique IDs | $r = i$ | Characterization of a sample |
| #Unique IDs * S/N of Reporters | $r = is$ | TMT multiplexing |
| #Unique IDs * S/N of MS/MS * S/N of Precursor | $r = imp$ | Label Free |
| #Completed Peaks * S/N of MS/MS | $r = cm$ | Emphasis on MS/MS Quantitation |
| #Unique IDs * #Experiments with Same ID | $r = i * n$ | Extra reward for same ID in multiple experiments |
| #Completed Peaks * S/N of MS/MS - #Not Completed Compounds | $r = cm - n$ | Penalty at end of experiment for not acquiring data for compounds |

## FIG. 9

FIG. 10

1000

A

1016 — GENERATING, BY THE SYSTEM, A REWARD INDICATOR RESULTING FROM THE RECOMMENDATION OF THE MASS SPECTROMETRY ACTION, OR FROM AN EXECUTION OF THE MASS SPECTROMETRY ACTION RECOMMENDED.

1018 — UPDATING, BY THE SYSTEM , ONE OR MORE WEIGHTS EMPLOYED BY THE NN OR MAKING AN ADJUSTMENT TO THE ACQUISITION METRICS, BASED ON THE REWARD INDICATOR.

1020 — UPDATING, BY THE SYSTEM, THE NN ACCORDING TO A SET OF REWARD INDICATORS AMORTIZED OVER TIME AND OBTAINED BASED ON A PLURALITY OF GENERATIONS OF A PLURALITY OF RECOMMENDATIONS, INCLUDING THE RECOMMENDATION TO PERFORM THE MASS SPECTROMETRY ACTION.

YES

SHOULD AN ADDITIONAL RECOMMENDATION BE GENERATED TO SATISFY THE METRIC OF INTEREST FOR THE COMPOUND?

1022

NO

END

B

FIG. 11

1200

1202 — IDENTIFYING, BY A SYSTEM OPERATIVELY COUPLED TO A PROCESSOR , A COMPOUND AND A SECOND COMPOUND FROM A MASS SPECTROMETRY SPECTRUM DEFINED BY FIRST DATA.

1204 — COMPARING, BY THE SYSTEM, FIRST DATA FOR THE COMPOUND TO AN INPUT DATASET COMPRISING ONE OR MORE ACQUISITION METRICS AND ONE OR MORE ASSOCIATED SCORES THAT ARE ASSOCIATED WITH THE ONE OR MORE ACQUISITION METRICS, FOR THE COMPOUND.

D

1206 — GENERATING, BY THE SYSTEM, A DATASET MATRIX FOR THE COMPOUND, BASED ON THE INPUT DATASET, AND COMPRISING THE ONE OR MORE ASSOCIATED SCORES, WHEREIN THE ONE OR MORE ASSOCIATED SCORES DEFINE PROBABILITIES THAT ONE OR MORE THRESHOLDS CORRESPONDING TO THE ONE OR MORE ACQUISITION METRICS WILL BE SATISFIED BY ONE OR MORE ADDITIONAL MASS SPECTROMETRY ACTIONS PERFORMED FOR THE COMPOUND.

1208 — GENERATING, BY THE SYSTEM, AN ASSOCIATED SCORE AS A NUMBER BETWEEN 0 AND 1.

1210 — BASED ON THE COMPARISON AND ON AN OBTAINED METRIC OF INTEREST ASSOCIATED WITH THE COMPOUND, AND EMPLOYING A NEURAL NETWORK TRAINED ON THE INPUT DATASET, GENERATING, BY THE SYSTEM, A FIRST RECOMMENDATION TO PERFORM A MASS SPECTROMETRY ACTION FOR THE COMPOUND.

1212 — GENERATING, BY THE SYSTEM, THE FIRST RECOMMENDATION BASED ON HISTORICAL DATA DEFINING ONE OR MORE RESULTS OF ACQUISITION OF THE COMPOUND CAUSED BY MASS SPECTROMETRY ANALYSIS OF THE COMPOUND.

C

**FIG. 12**

1200

C

PRIOR TO THE EMPLOYING OF THE NEURAL NETWORK TO GENERATE THE RECOMMENDED MASS SPECTROMETRY ACTION, CORRELATING, BY THE SYSTEM, THE INPUT DATASET TO THE METRIC OF INTEREST.

1214

GENERATING, BY THE SYSTEM, THE FIRST RECOMMENDED MASS SPECTROMETRY ACTION COMPRISING USE OF A MASS SPECTROMETRY DEVICE TO DIRECTLY OR INDIRECTLY OBTAIN ACQUISITION OF THE COMPOUND.

1215

SELECTING, BY THE SYSTEM, THE FIRST RECOMMENDED MASS SPECTROMETRY ACTION COMPRISING A FRAGMENTED ACQUISITION THAT ACQUIRES THE COMPOUND.

1216

SELECTING, BY THE SYSTEM, THE FIRST RECOMMENDED MASS SPECTROMETRY ACTION FROM AN ACTION DATABASE OF AVAILABLE MASS SPECTROMETRY ACTIONS, WHICH AVAILABLE MASS SPECTROMETRY ACTIONS ARE CAPABLE OF BEING PERFORMED USING ONE OR MORE MASS SPECTROMETRY DEVICES COMMUNICATIVELY COUPLED TO THE SYSTEM.

1217

GENERATING, BY THE SYSTEM, IN PARALLEL, THE FIRST RECOMMENDATION AND A SECOND RECOMMENDATION FOR THE SECOND COMPOUND.

1218

EVALUATING, BY THE SYSTEM, THE RECOMMENDED MASS SPECTROMETRY ACTION, RESULTING IN A REWARD INDICATOR OBTAINED BY THE SYSTEM, WHEREIN THE REWARD INDICATOR IS EMPLOYED TO UPDATE ONE OR MORE WEIGHTS EMPLOYED BY THE NEURAL NETWORK.

1220

SHOULD AN ADDITIONAL RECOMMENDATION BE GENERATED TO SATISFY THE METRIC OF INTEREST FOR THE COMPOUND?

1222

YES

D

NO

END

**FIG. 13**

1400

1402 — GENERATING, BY A SYSTEM OPERATIVELY COUPLED TO A PROCESSOR, AN INPUT DATASET, COMPRISING ONE OR MORE ACQUISITION METRICS AND ONE OR MORE ASSOCIATED SCORES THAT ARE ASSOCIATED WITH THE ONE OR MORE ACQUISITION METRICS, CORRESPONDING TO A TYPE OF COMPOUND.

1404 — GENERATING, BY THE SYSTEM THE INPUT DATASET COMPRISING THE ONE OR MORE ASSOCIATED SCORES COMPRISE PROBABILITIES THAT ONE OR MORE THRESHOLDS CORRESPONDING TO THE ONE OR MORE ACQUISITION METRICS WILL BE SATISFIED BY A MASS SPECTROMETRY ACTION PERFORMED FOR THE TYPE OF COMPOUND.

1406 — TRAINING, BY THE SYSTEM, A NEURAL NETWORK ON THE INPUT DATASET.

F

1408 — GENERATING, BY THE SYSTEM, A RECOMMENDED MASS SPECTROMETRY ACTION FOR OBTAINING SECOND DATA ON A COMPOUND OF THE TYPE OF COMPOUND BY EMPLOYING THE NEURAL NETWORK TO COMPARE FIRST DATA FOR THE COMPOUND TO THE INPUT DATASET ASSOCIATED WITH THE TYPE OF COMPOUND.

1410 — GENERATING, BY THE SYSTEM, THE RECOMMENDED MASS SPECTROMETRY ACTION BASED ON A METRIC OF INTEREST FOR THE TYPE OF COMPOUND, WHICH METRIC OF INTEREST IS PROVIDED BY A USER ENTITY TO TAILOR FUNCTIONING OF THE NEURAL NETWORK.

1412 — SPECIFYING, BY THE SYSTEM, AN AMOUNT OF A RESOURCE TO EMPLOY FOR THE RECOMMENDED MASS SPECTROMETRY ACTION, BASED ON HISTORICAL DATA DEFINING ACQUISITION OF THE COMPOUND CAUSED BY MASS SPECTROMETRY ANALYSIS OF THE TYPE OF COMPOUND.

E

# FIG. 14

1400

E

1414 — SPECIFYING, BY THE SYSTEM, THE COMPOUND, OR A FRAGMENTED COMPOUND IDENTIFIED FROM A MASS SPECTROMETRY SPECTRUM, AS A TARGET OF THE RECOMMENDED MASS SPECTROMETRY ACTION, WHEREIN THE FIRST DATA DEFINES THE MASS SPECTROMETRY SPECTRUM FOR THE COMPOUND.

1416 — SPECIFYING, BY THE SYSTEM, A SET OF TWO OR MORE TARGETS FOR WHICH TO OBTAIN THE SECOND DATA BY PERFORMANCE OF THE RECOMMENDED MASS SPECTROMETRY ACTION.

1418 — GENERATING, BY THE SYSTEM, THE RECOMMENDED MASS SPECTROMETRY ACTION COMPRISING A FRAGMENTED SCAN THAT IS A MASS SPECTROMETRY/MASS SPECTROMETRY (MS2) ACQUISITION FOR THE COMPOUND OR A MASS SPECTROMETRY/ MASS SPECTROMETRY/MASS SPECTROMETRY (MS3) ACQUISITION FOR THE COMPOUND.

SHOULD AN ADDITIONAL RECOMMENDATION BE GENERATED TO SATISFY THE METRIC OF INTEREST FOR THE COMPOUND?

1420

YES

F

NO

END

**FIG. 15**

1600

**FIG. 16**

1700

1720

1710

REMOTE
COMPONENT(S)

LOCAL
COMPONENT(S)

REMOTE
DATA
STORE(S)

COMMUNICATION
FRAMEWORK

LOCAL
DATA
STORE(S)

1750

1730

1740

FIG. 17

FIG. 18

# EUROPEAN SEARCH REPORT

Europäisches Patentamt
European Patent Office
Office européen des brevets

**Application Number**

EP 24 20 6977

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PELLETIER ALEXANDER R. ET AL: "MealTime-MS: A Machine Learning-Guided Real-Time Mass Spectrometry Analysis for Protein Identification and Efficient Dynamic Exclusion", JOURNAL OF THE AMERICAN SOCIETY FOR MASS SPECTROMETRY, vol. 31, no. 7, 8 June 2020 (2020-06-08), pages 1459-1472, XP093255522, US ISSN: 1044-0305, DOI: 10.1021/jasms.0c00064 Retrieved from the Internet: URL:https://pubs.acs.org/doi/pdf/10.1021/jasms.0c00064> * the whole document * | 1-11 | INV.<br>G16C20/20<br>G16C20/70<br><br>ADD.<br>H01J49/00<br>G01N33/68 |
| A | WALLMANN GEORG ET AL: "Data-Driven Optimization of DIA Mass Spectrometry by DO-MS", JOURNAL OF PROTEOME RESEARCH, vol. 22, no. 10, 11 September 2023 (2023-09-11), pages 3149-3158, XP093255442, ISSN: 1535-3893, DOI: 10.1021/acs.jproteome.3c00177 Retrieved from the Internet: URL:https://pubs.acs.org/doi/pdf/10.1021/acs.jproteome.3c00177> * the whole document * | 1-11 | |
| A | WO 2018/213112 A1 (BIOANALYTIX INC [US]) 22 November 2018 (2018-11-22) * the whole document * | 1-11 | TECHNICAL FIELDS SEARCHED (IPC)<br><br>G16C<br>H01J<br>G06N<br>G01N |

-/--

The present search report has been drawn up for all claims

| Place of search | Date of completion of the search | Examiner |
|---|---|---|
| The Hague | 3 March 2025 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 24 20 6977

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2022/043920 A1 (WATERS TECHNOLOGIES IRELAND LTD [IE]) 3 March 2022 (2022-03-03) * the whole document * | 1-11 | |
| A | HEIL LILIAN R. ET AL: "Dynamic Data-Independent Acquisition Mass Spectrometry with Real-Time Retrospective Alignment", ANALYTICAL CHEMISTRY, vol. 95, no. 32, 1 August 2023 (2023-08-01), pages 11854-11858, XP093255434, ISSN: 0003-2700, DOI: 10.1021/acs.analchem.3c00903 Retrieved from the Internet: URL:https://pubs.acs.org/doi/pdf/10.1021/acs.analchem.3c00903> * the whole document * | 1-11 | |

TECHNICAL FIELDS
SEARCHED      (IPC)

The present search report has been drawn up for all claims

| Place of search | Date of completion of the search | Examiner |
|---|---|---|
| The Hague | 3 March 2025 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 6977

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2025

| Patent document cited in search report | Publication date | Patent family member(s) | | Publication date |
|---|---|---|---|---|
| WO 2018213112 A1 | 22-11-2018 | NONE | | |
| WO 2022043920 A1 | 03-03-2022 | CN | 116438625 A | 14-07-2023 |
| | | EP | 4205161 A1 | 05-07-2023 |
| | | US | 2022084802 A1 | 17-03-2022 |
| | | WO | 2022043920 A1 | 03-03-2022 |

For more details about this annex : see Official Journal of the European Patent Office, No. 12/82